# EUROPEAN PATENT APPLICATION

(11) **EP 2 502 932 A1**
(43) Date of publication of application: **26.09.2012**
(21) Application number: 11159920.5
(22) Date of filing: 25.03.2011
(51) Int. Cl.: C07K 14/40, C12N 9/02, C12N 9/88, C12P 7/64

(54) **Novel beta-oxidation pathway and hemicascomycetes yeast mutants**

(71) Applicant: Université Bordeaux Segalen, 33076 Bordeaux Cedex (FR); Centre National de la Recherche Scientifique CNRS, 75794 Paris Cedex 16 (FR); Centre Hospitalier Universitaire de Bordeaux, 33404 Talence (FR)
(72) Inventor: Noel, Thierry, 33800 Bordeaux (FR); Accoceberry, Isabelle, 33000 Bordeaux (FR); Bessoule, Jean Jacques, 33000 Bordeaux (FR); Manon, Stephen Thierry, 33200 Bordeaux (FR); Gabriel, Frederic, 33700 Merignac (FR)
(74) Representative: Lecca, Patricia S.

(57) **Abstract**

The present invention relates generally to the fields of microbiology and biotechnology. More precisely, the present invention relates to isolated biologically pure hemiascomycetes yeast strain comprising unique combination of metabolic pathways for the catabolism or degradation of fatty acid and other substrates. The present invention also provides a method of increasing the production of commodity chemicals using the isolated biologically pure hemiascomycetes yeast strain. The present invention further provides novel mutants that can yield improved production of such commodity chemicals.

## Description

### Technical field of the invention

The present invention relates generally to the fields of microbiology and biotechnology. More precisely, the present invention relates to isolated biologically pure hemiascomycetes yeast strain comprising unique combination of metabolic pathways for the catabolism or degradation of fatty acid and other substrates. The present invention also provides a method of increasing the production of commodity chemicals using the isolated biologically pure hemiascomycetes yeast strain. The present invention further provides novel mutants that can yield improved production of such commodity chemicals.

**Background of the invention**

Cellular fatty acid degradation occurs via the β-oxidation pathway in all organisms. So far it has been established that there are two different β-oxidation systems in eukaryotes: the β-oxidation located in mitochondria for mammals and some filamentous fungi and the β-oxidation system located in peroxisomes for plants, fungi and animals.

Fatty acid beta-oxidation begins with the addition of coenzyme A to a fatty acid, and occurs by successive cycles of reactions during each of which the fatty acid is shortened by a two-carbon fragment removed as acetyl coenzyme A, generating trans-2,3 hydroxyl, and 3-keto intermediates, until only two or three carbons remain (as acetyl-CoA or propionyl-CoA respectively). The proteins involved in the mitochondrial β-oxidation and in the peroxisomal β-oxidation are however different. Multifunctional proteins (MFPs) or multifunctional enzymes (MFEs) are involved in the peroxisomal β-oxidation pathway, whereas β-oxidation consists of monofunctional enzymes.

The peroxisomal β-oxidation process begins with oxidation of the acyl-CoA substrate into trans-2-enoyl-CoA by Acyl-CoA oxidase, namely Fox1p/Pox1p. It has been demonstrated that *Pox1*Δ yeasts are unable to grow on fatty acids as sole carbon atoms. Then the peroxisomal β-oxidation proceeds from trans-2-enoyl-CoA to 3-ketoacyl-CoA *via* the (3R)-hydroxyacyl-CoA ester intermediates. In the yeast oxidation system, the second and third reactions of the β-oxidation cycle are catalyzed by the same enzymes called Mfe2p, Fox2p or again Pox2p, which contains both the 3-hydroxyacyl-CoA and 2-enoyl-CoA hydratase activities. The 2-enoyl-CoA hydratase converts the trans-2-enoyl CoA esters into (3R)-hydroxyacyl-CoA esters, whereas the hydratase 2 produces the 3-ketoacyl-CoA. This enzyme was first isolated from *Candida tropicalis* and comprise a duplicated domain organization in its N-terminal region, which contains two deshydrogenase active domains A and B. Domain A was demonstrated to have highest activity with long and medium chain substrates, whereas domain B has the highest activity with short-chain substrates. The C-terminal region of the Fox2p enzyme contains the 2-enoyl-CoA hydratase 2 activity. Hiltunen et al. (JBC, Vol. 267, No. 10, April 5, 1992, pp 6646-6653) showed that fatty acid catabolism in yeast was mainly based on the activity of Fox2p and that disruption of *FOX2* resulted in the inability of yeast cells to grow on fatty acids as their sole carbon source. At the next reaction of the β-oxidation cycle the ketoacyl-CoA intermediate undergoes thiolytic cleavage by a 3-ketoacyl-CoA thiolase, namely Pot1p/Fox3p. The Pot1p/Fox3p is a dimeric protein with a subunit size of 45kDa. A single subunit comprises three domains: two core domains, and a loop domain of 120 residues. The active site of yeast thiolase is shaped by residues from the two core domains and surrounded by the loop domain. The products of this last step are acetyl-CoA and a C2-shortened acyl-CoA, which acts as substrate for Pox1 p/Fox1 p for an additional cycle. The acetyl-CoA which is produced by peroxisomal beta oxidation is then used in the glyoxilic cycle, thereby allowing the transformation of acetyl-CoA into oxaloacetate. These reactions are catalyzed by two enzymes: isocitrate lyase (Icl1p) and malate synthase (Mls1p) which permits the use of two carbon atoms such as acetate, in the neoglucogenese.

In contrast with the well-established and accepted dogma that β-oxidation in yeasts was restricted in the peroxisomes, whereas the β-oxidation in mammals and some filamentous fungi occurs both in mitochondria and peroxisomes, the Applicant has demonstrated in fact that some hemiascomycetous yeast also housed β-oxidation in mitochondria, as for mammals and some filamentous fungi. The Applicant further demonstrated that the peroxisomal β-oxidation pathway was in fact more complex for some hemiascomycetes, and surprisingly revealed the existence of *FOX2* independent β-oxidation, in addition to the well-known *FOX2* dependent peroxisomal dependent pathway.

These new developments greatly contribute to the use of these hemiascomycetes yeast cells not only as a favorite model organism in biological research, but also as a target for metabolic engineering in preparation for novel biotechnological applications.

Indeed, hemiascomycetes yeasts are industrially important and have been used to produce many of the so-called commodity chemicals, such as for example, fatty acids, acyl glycerides, bioflavours, aromas, fragrances, bio-oil, etc... These commodity chemicals are widely used in food industry, animal nutrition, cosmetics, and in the pharmaceutical field.

In the end of the 1980s, the consumer has developed a "chemophobia" attitude towards chemical or synthetic (even nature-identical) compounds, especially when related to his food and home-care products. The interest of consumers for natural products thus rose. The word "natural" is defined legally by American and European regulations and a substance can be considered as natural when it comes from a plant, animal or microbial origin with a physical, microbial or enzymatic process. Therefore, biotechnological routes may be, if they exclude any chemical steps, a way to get natural products. As a result, the industry has devoted considerable time and effort to develop methods for the production of natural components which have been showed to be useful in various industries.

By way of examples, the engineered microorganisms are particularly useful for the production of polyunsaturated fatty acids (PUFAs), such as linoleic acid or linolenic acid, and more generally w-3 fatty acids (omega-3 fatty acids) and w-6 fatty acids (omega-6-fatty acids) such as. These are indeed essential fatty acids for mammals, and thus are supplemented to food of humans and animals. These may be derived from fish oils, like herring, mackerel, sardine or salmon, but requires complex extraction and purification procedures, which, especially if carried out on an industrial scale, are expensive, laborious and tend to pollute the environment. Thus, there is a need for improved production of PUFAs in a pure and cost effective form, which also allow large scale and automated process formats.

The engineered microorganisms are also very useful for production of bio-flavours, fragrances and aromas. It is generally recognized in the industry that a flavour compound having been prepared by microbial processes can be designated as a natural product and therefore have an important place in the commercialization of products containing them. The market for these chemicals has been quite dynamic since a long time, and currently stands at around 7 billion USD. In the recent past, many pathways were investigated and about 400 natural aroma compounds were proposed to the market.

Numerous fungal and yeast strains, such as *Candida tropicalis* or *Yarrowia lipolytica,* have been used to synthesize various commodity substances. Several mutant strains of *Candida* have been developed by strain selection to produce several substances. The yields of this biotransformation are however often disappointingly low, and are rarely above 100 mg/L, making these processes economically unattractive.

Therefore, in spite of all the prior work, a bottleneck still exists in the lack of complete knowledge about the biochemical pathways, the enzymes and the metabolic regulation involved for obtaining high yields of commodity chemicals. A better understanding of the fungal biochemistry and enzymes involved, metabolic regulation and genetic modification are primordial to improve yields, in addition to the application of novel fermentation technology.

As indicated above, it has been surprisingly discovered by the Applicant that there exists an alternate peroxisomal pathway for catabolism which is *FOX2* independent, as well as a mitochondrial catabolic pathway functional in hemiascomycetes yeasts. Novel isolated biologically pure yeast strains have been identified and novel mutant strains have been selected on the basis of their unique metabolic pathways to achieve desirable production of commodity chemical compounds with improved production capacities.

### Summary of the invention

The present invention relates to an isolated, biologically pure hemiascomycetes yeast strain comprising (i) at least one Fox2p independent peroxisomal β-oxidation pathway (ii) at least one Fox2p dependent peroxisomal β-oxidation pathway, and (iii) one Fox2p dependent mitochondrial β-oxidation pathway, wherein such strain still does grow on fatty acid carbon source when rendered non functional for the *FOX2* gene.

The present invention also relates to the use of the isolated, biologically pure hemiascomycetes yeast strain, genetic mutants of these hemiascomycetes yeast strains as well as methods of producing various commodity chemicals, including like fatty acids, PUFA, saturated and unsaturated lipids, phospholipids, phospholipids esters, triacylglycerols, biofuels and/or biodiesel, fragrances and flavouring substances, such as lactones, esters, aldehydes, terpenes, and derivatives thereof, etc...

### Brief description of the figures

**Figure 1** : shows the stragegy used for the construction of an *OLE2* deletion cassette by overlapping PCR, and for the selection of an *ole2*Δ mutant strain after homologous recombination and replacement of the wild *OLE2* allele of the recipient strain by the deletion cassette carrying *URA3.*

**Figure 2** : shows the stragegy used for the construction of an *ECH1* deletion cassette by overlapping PCR, and for the selection of an *ech1*Δ mutant strain after homologous recombination and replacement of the wild *ECH1* allele of the recipient strain by the deletion cassette carrying *URA3.*

**Figure 3****:** shows the nucleotide sequence of the *FOX2* gene (SEQ ID NO: 1) in *Clavispora lusitaniae.*

**Figure 4****:** shows the amino acid of Fox2p protein (SEQ ID NO: 2) in *Clavispora lusitaniae.*

**Figure 5****:** shows the nucleotide sequence of the *PAX1* gene (SEQ ID NO: 3) in *Clavispora lusitaniae.*

**Figure 6****:** shows the amino acid of Pax1 p (SEQ ID NO: 4) in *Clavispora lusitaniae.*

**Figure 7****:** shows the nucleotide sequence of the *OLE2* gene (SEQ ID NO: 5) in *Clavispora lusitaniae.*

**Figure 8****:** shows the amino acid of Ole2p (SEQ ID NO: 6) in *Clavispora lusitaniae.*

**Figure 9****:** shows the nucleotide sequence of the *ECH1* gene (SEQ ID NO: 7) in *Clavispora lusitaniae.*

**Figure 10****:** shows the amino acid of the Ech1 p (SEQ ID NO:8) in *Clavispora lusitaniae.*

### Detailed description

The present invention relates to an isolated, biologically pure hemiascomycetes yeast strain comprising three distinct metabolic pathways for the catabolism of fatty acid: (i) at least one pathway is a peroxisomal β-oxidation *FOX2* independent pathway, (ii) at least one peroxisomal β-oxidation *FOX2* dependent pathway, and (iii) a mitochondrial β-oxidation *FOX2* dependent pathway.

The isolated pure hemiascomycetes yeast strain according to the present invention does not grow on fatty acid carbon source when rendered non functional for the *FOX2* gene. By "isolated" is meant material that is substantially or essentially free from components that normally accompany it in its native state.

The term "yeast" refers to an organism of the kingdom Fungi, and preferably, is directed towards any organism of the phylum Ascomycota and most preferably is directed towards any organism of the class Hemiascomycetes. The ascomycetes generally occur in two significantly distinctive types: those which form a filamentous mycelium, the "euascomycetes" and those which do not, the "hemiascomycetes". Hemiascomycetes as referred to herein correspond to a group including *yeast* species sharing a small genome size and a low frequency of introns.

More precisely, hemiascomycetes yeast strains according to the invention include strains of the genus *Candida, Debaryomyces, Clavispora, Metschnikowia, Kluyveromyces, Lodderomyces, Pichia, Hansenula, Lipomyces, Yarrowia, and Geotrichum.* By way of examples, yeasts of the genus *Candida* include *C*. *tropicalis, C. parapsilosis, C. orthopilosis, C. metapsilosis, C. norvegensis, C. antartica, C. dubliniensis, C. haemulonii, C. kefyr, C. krusei, C. lusitaniae, C. maltose,* and *C. oleophila.* Yeasts of the genus *Debaryomyces* include *D. hansenii* and *D*. *castelli.* Yeasts of the genus *Clavispora* include *C. lusitaniae and C. opuntiae.* Yeasts of the genus *Metschnikowia* include for example *M*. *pulcherrima.* Yeasts of the genus *Kluyveromyces* include *K. lactis, K. marxianus, K. yarrowii,* and *K. wickerhamii.* Yeasts of the genus *Lodderomyces* include for example *L*. *elongisporus.* Yeasts of the genus *Pichia* include *P. guilliermondii, P. anomala, P. fabianii, P. kluyveri, P. norvegensis, P. ohmeri, P. pastoris* and *P. angusta.* Yeasts of the genus *Hansenula* include for example *H. polymorpha.* Yeasts of the genus *Lipomyces* include for example *L. starkeyi, L. orientalis, L. yarrowii, and L. japonica.* Yeasts of the genus *Yarrowia* include for example *Y. lipolytica.* Yeasts of the genus *Geotrichum* include for example *G*. *candidum, G. clavatum,* and *G. fici.*

Peroxisomes are single-membrane-bound organelles that not only contain the enzymes for the β-oxidation of fatty acids but also can be involved in a variety of other metabolic pathways, such as the inactivation of toxic substrates (H₂O₂-based respiration), the synthesis of etherphospholipids (in mammals), and the breakdown of purines and amino acids. Peroxisome biogenesis is a conserved process among eukaryotes, involving the concerted action of at least 32 proteins (peroxins) that are encoded by *PEX* genes.

As indicated above, the present invention is contrary to the well-established dogma that β-oxidation in yeasts is restricted in the peroxisomes, whereas the β-oxidation in mammals and in some filamentous fungi which occurs both in mitochondria and peroxisomes, the Applicant has demonstrated in fact also housed in mitochondria, as for mammals. The Applicant further demonstrated that the peroxisomal β-oxidation pathway was in fact more complex for some hemiascomycetes, and surprisingly revealed the existence of *FOX2* independent β-oxidation, in addition to the well-known *FOX2* dependent peroxisomal dependent pathway.

The novel isolated biologically pure yeast strains are surprisingly capable of growing and producing desired molecules via a peroxisomal β-oxidation *FOX2* independent pathway, while having their β-oxidation *FOX2* dependent pathways blocked. The isolated, biologically pure hemiascomycetes yeast strains have been characterized as having, in addition to the peroxisomal β-oxidation *FOX2* dependent pathway, at least one peroxisomal β-oxidation *FOX2* independent pathway, as well as a mitochondrial β-oxidation *FOX2* dependent pathway.

According to the present invention, the isolated biologically pure hemiascomycetes yeast strains as described above may comprise one or more pathways, *i.e*., either the peroxisomal β-oxidation *FOX2* dependent pathway and/or the mitochondrial β-oxidation *FOX2* dependent pathway, disrupted or enhanced by at least one genetic modification.

Preferably, the isolated, biologically pure hemiascomycetes yeast strains belong to the genus *Candida,* and more preferably to the species *Candida lusitaniae,* and comprise one or more genetic modifications which may disrupt or enhance the mitochondrial *FOX2* dependent pathway and/or the peroxisomal *FOX2* dependent pathway.

By "genetic modifications" is meant genetic mutations, total or partial gene deletions or excisions, gene disruptions or integrations, and/or gene duplications. Such genetic modifications are accomplished by genetic engineering methods which are well known in the art.

Such genetic modifications may be performed for example by mutagenesis or other genetic engineering methods well known methods in the art, followed by appropriate selection or screening to identify the desired mutants. Method of deletion may be preferably performed according to the strategy described in El-Kirat-Chatet K et al. (Yeast. 2010 Nov 24).

According to a preferred embodiment of the present invention, the isolated biologically pure hemiascomycetes yeast strains comprise at least one more genetic modification of the *FOX2* gene. As described above, *FOX2* gene is rendered non functional and the strains comprise a total or partial gene deletion, excision, disruption insertion or integration within the *FOX2* gene. Preferably, *FOX2* gene is partially or totally deleted. Most preferably, the *FOX2* gene is rendered non functional by insertion of another gene of an unrelated metabolic pathway and/or by an antibiotic resistance gene (AB^{R}).

Alternatively, the isolated biologically pure hemiascomycetes yeast strains comprise one or more duplication of the *FOX2* gene to enhance the peroxisomal *FOX2* dependent pathway.

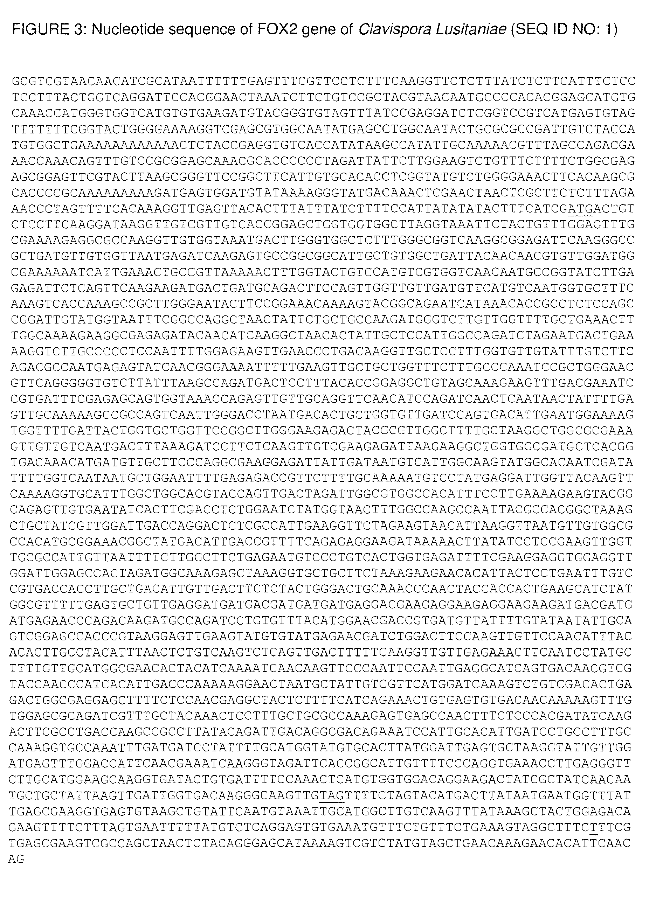

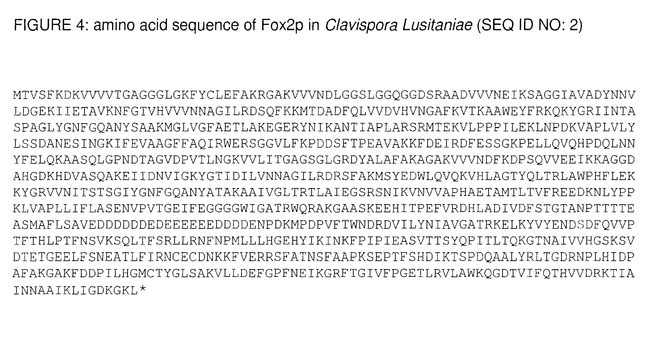

As indicated above enzymes-encoding genes involved in fatty acid oxidation include *FOX1* (Fatty acid oxidation gene 1)*lPOX1* (Fatty-acyl coenzyme A oxidase 1), *FOX2* (Fatty acid oxidation gene 2)/*POX2* (Fatty-acyl coenzyme A oxidase 2). Fatty acid oxidation-2 gene (hereinafter FOX2 gene) encodes the second enzyme of the β-oxidation pathway. Fox2p is the enzyme with dual activity: 3-hydroxyacyl-CoA dehydrogenase and enoyl-CoA hydratase (Seem Hiltunen, et al., J. Biol. Chem. 267:6646-53 (1992)). The gene encoding Fox2p of *C*. *lusitaniae* has been isolated and sequenced and encodes a 903 amino acid protein. Nucleotide and amino acid sequences are as set forth in Figures 3 and 4, respectively. 3)/*POT1*/ *FOX3* (3-ketoacyl-CoA thiolase) encodes the third enzyme of the β-oxidation pathway;

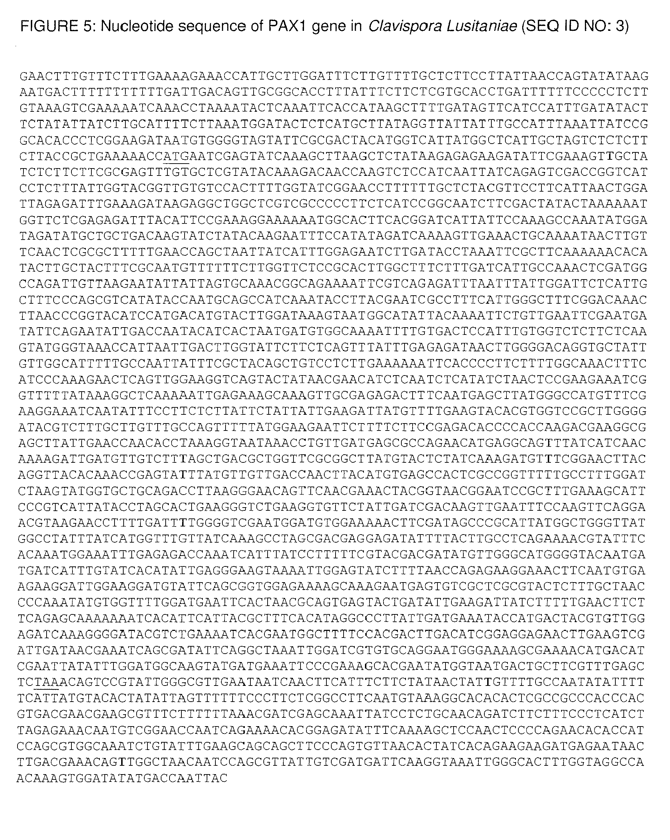

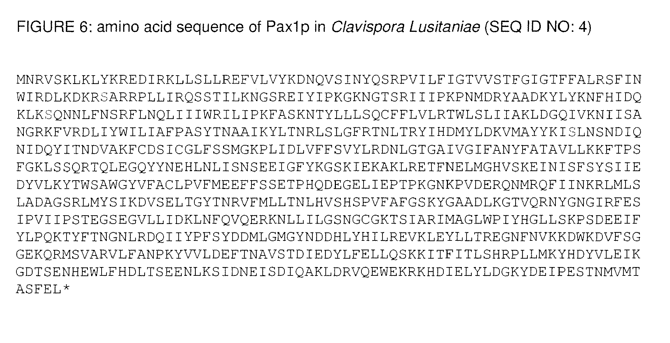

The isolated biologically pure hemiascomycetes yeast strains according to the present invention may further comprise or a genetic modification of the P*XA1* gene. Preferably, such strains comprise a partial or total deletion of the *PXA1* gene. Yeast contains two ALDP (Adrenoleukodystrophy Protein) homologues: *PXA1* (Peroxisomal ABC-transporter 1), also known as *LPI1, PAT2, SSH2* or *PAL1,* and *PXA2,* also known as *PAT1* (Protein Associated with Topoisomerase 1). The Pxa1 p-Pxa2p transporter is reported to bear sufficient similarity to the human adrenoleukodystrophy protein ALDP. During β-oxidation, the two peroxisomal half ABC transporters Pxa1 p and Pxa2p form a heterodimeric complex in the peroxisomal membrane. The gene of C. lusitaniae encoding Pax1 p has been isolated and sequenced and encodes a 809 amino acid protein. Nucleotide and amino acid sequences are as set forth in Figures 5 and 6, respectively.

The isolated biologically pure hemiascomycetes yeast strains and genetically modified strains as described above are capable of accumulating lipids and are thus particularly advantageous for several biotechnology applications, particularly for the production of commodity chemicals. The present invention thus also relates to the use of the biologically pure strains and mutants thereof as well as to methods of producing of commodity chemicals.

"Commodity chemical" as used herein includes any lipids that can be produced either directly or as a by-product and can be used in various industries. These include for example free fatty acids, long chain fatty acids, saturated, mono or polyunsaturated fatty acids (PUFAs), triacylglycerols (TAGs), phospholipids, phospholipids esters, phospholipids mono and/or diesters, biofuels/biodiesels, lipid derived fragrances and/or flavourings (bioflavours).

The term "fatty acids" refers to long chain aliphatic acids (alkanoic acids) of varying chain lengths, from about C₁₂ to C₂₄, and preferably having chain lengths from about between C₁₆ to C₂₄. These may be saturated fatty acids, unsaturated fatty acids, monounsaturated fatty acids, and polyunsaturated fatty acids.

PUFAs refer to fatty acids comprising at least 18 carbon atoms and at least two double bonds, more preferably the term relates to omega-3 and omega-6 fatty acids comprising at least 18 carbon atoms, even more preferably the term relates to linoleic acid, conjugated linoleic acid (CLA), gamma-linolenic acid (GLA), dihomo-gamma-linolenic acid (DGLA), arachidonic acid (ARA), alpha-linolenic acid (ALA), stearidonic acid (STA), eicosatetraenoic acid (ETA), eicosapentaenoic acid, docosapentaenoic acid (DPA) or docosahexaenoic acid (DHA). The terms "omega-3" and "omega-6" relates to double bonds in a fatty acid, which are located on the 3^{rd} or 6^{th} carbon-carbon bond, respectively, counting from the w, (methyl carbon) end of the fatty acid molecule.

Preferably, the fatty acids synthesized by the mutated strains according to the present invention are polyunsaturated fatty acids such as linoleic acid, conjugated linoleic acid, gamma-linolenic acid, dihomo-gamma-linolenic acid, arachidonic acid, alpha-linolenic acid, stearidonic acid, eicosatetraenoic acid, eicosapentaenoic acid, docosapentaenoic acid or docosahexaenoic acid.

TAGs refer to the lipids composed of three fatty acyl resides esterified to a glycerol molecule. TAGs can contain long chain PUFAs and saturated fatty acids, as well as shorter chain saturated and unsaturated fatty acids.

The isolated biologically pure hemiascomycetes yeast strains and genetically modified strains are preferably used to produce long chain fatty acids, preferably unsaturated, mono and/or polyunsaturated fatty acids (PUFAs), most preferably C₁₆-C₂₄ fatty acids, and most specifically are linoleic acid and/or γ-linoleic acid.

Lipid derived fragrances and/or flavourings are natural fragrances, natural aromas, or bioflavours and refer to compounds resulting from the extraction of natural materials and/or to be transformed by natural means. They include for example terpenes, sesquiterpenes, aldehydes, esters, lactones and derivatives thereof.

The terms "terpenes and derivatives" refer to a large and varied class of organic compounds derived biosynthetically from units of isoprene, which has the molecular formula C₅H₈. The basic molecular formulae of terpenes are multiples of (C₅H₈)ₙ,These may be selected from Hemiterpenes C₅H₈, Monoterpenes C₁₀H₁₆, Sesquiterpenes C₁₅H₂₄, Diterpenes C₂₀H₃₂, Sesterterpenes C₂₅H₄₀, Triterpenes C₃₀H₄₈, Tetraterpenes C₄₀H₆₄, Polyterpenes (C₅H₈)n. Derivatives include and are not restricted to iridoids, tetrahydrocannabinol, guaianolides, pseudoguaianolides, eudesmanolides, eremophilanolides, and xanthanolides etc... Flavour-terpenes compounds include for example citronellol, geraniol, linalol nerol, and α-terpineol.

Lactones are a family of flavors and fragrances. Lactones are cyclic esters of primarily γ- and δ-hydroxy acids and are produced via a key step is the hydroxylation of fatty acids. Most of the other lactones are also encountered in fermented products but are far more difficult to produce. Macrocyclic lactones are used for their musk notes in cosmetics and perfumes and their production also uses a yeast metabolic pathway. Depending on the compounds, they exhibit fruity and oily properties with peach, apricot, milky or coconut notes. Fruit and/or milk flavourings or fragrances generally comprise saturated and unsaturated δ- and γ-lactones.

By way of example, peach-like fragrances comprise γ-decalactone (4-decanolide), 4-hydroxy-cis-dodecanoic acid-γ-lactone, δ-decalactone, and/or γ-octalactone, whereas coconut milk-like fragrances comprise 4-dodecanolide and/or 4-octanolide.

δ-decalactone and δ-dodelactone are highly appreciated flavouring compounds occurring in minute quantities in milk products and are responsible for bringing out the typical flavour in these products. Due to their low natural occurrence and their highly appreciated organoleptic characteristics, there exists a strong need for processes capable of providing industrial quantities of these lactones.

γ-Decalactone is present naturally in many fruits and fermented products. It is particularly important in the formulation of peach, apricot, and strawberry flavours. Microbial processes to produce this compound have been described, although the metabolic pathways involved were not completely defined so far.

Other lactones of interest may include for examples γ-valerolactone, γ-hexalactone, γ-heptalactone, γ-nonalactone, γ-undecalactone, γ-dodecalactone, γ-tridecalactone, γ-tetradecalactone, δ-hexalactone, δ-heptalactone, δ-octalactone, δ-nonalactone, δ-undecalactone, δ-dodecalactone, δ-tridecalactone, decanolides and δ-tetradecalactone.

The present invention is directed to strategies for increasing the production of lactones. In particular, the method of the present invention may be used for the production of various lactones, such as δ-decalactone, and/or γ-octalactone, which may be specifically produced by culturing the genetically modified strain according to the current invention in a medium enriched with vegetable oil and/or ricinoleic acid substrates, preferably castor oil, a castor oil hydrolysate, and the like.

δ-decalactone may alternatively be produced by comprising culturing the strains according to the present invention in a medium enriched with massoi bark oil and/or hydroxypalmitic acid substrates like 11-hydroxypalmitic acid and/or ethyl 11-hydroxypalmitate.

Flavour-esters include for example volatile branched acetates, such as 3-methylbutyl-acetate which has a banana aroma, isobutylacetate, and 2-methylbutylacetate.

The recitation "biofuels/biodiesel" as used herein includes solid, liquid, or gas fuels derived, at least in part, from a biological source, such as a yeast cell. Trans-esterification of lipids yields long-chain fatty acid esters useful as biodiesel. For production of such biofuels, suitable known and commercially available media may be employed. The media may comprise suitable monosaccharide or oligosaccharide, biomass selected from marine biomass (kelp, giant kelp, sargasso, seaweed, algae, marine microflora, microalgae, and sea grass), vegetable/fruit/plant biomass, cellulose, hemicellulose, pectin, lignocellulose etc...

Furthermore, the strains as described above may comprise a genetic modification of one or more of desaturase genes. Desaturase genes encode polypeptides which can desaturate, *i*.*e*., introduce a double bond, in one or more fatty acid or precursors. It is usual to indicate activity of the desaturase by counting from the carboxyl end of the substrate. For example Δ5 desaturases desaturate a fatty acid between the fifth and sixth carbon atom numbered from the carboxyl-terminal end of the molecule and can catalyze the conversion of dihomo gamma linolenic acid (DGLA) to arachidonic acid (ARA), and/or eicosatetraenoic acid (ETA) to eicosapentaenoic acid (EPA).

Of particular interest herein are Δ6 desaturases that catalyze the conversion of linoleic acid (LA) to gamma linolenic acid (GLA) and/or α-linolenic acid (ALA) to stearidonic Acid (STA); Δ12 desaturases that catalyze the conversion of oleic acid to linoleic acid; and Δ15 desaturases that catalyze the conversion of linoleic acid (LA) to (α-linolenic acid) ALA and/or gamma linolenic acid (GLA) to stearidonic Acid (STA).

Other useful fatty acid desaturases include, for example: Δ8 desaturases that can desaturate eicosadienoic acid (EDA) to dihomo gamma linolenic acid (DGLA) and/or eicosatrienoic acid (EtrA) to eicosatetraenoic acid (ETA); Δ4 desaturases that catalyze the conversion of DPA to DHA; Δ-17 desaturases that catalyze the conversion of arachidonic acid (ARA) to eicosapentaenoic acid (EPA) and/or dihomo gamma linolenic acid (DGLA) to eicosatetraenoic acid (ETA).

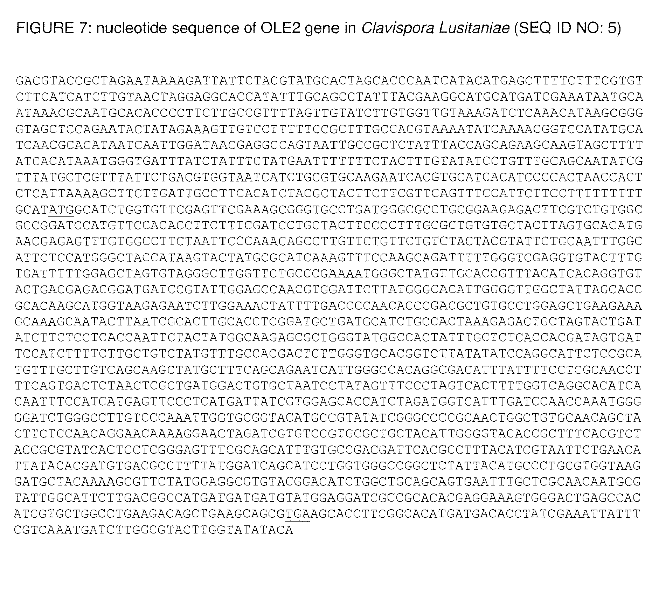

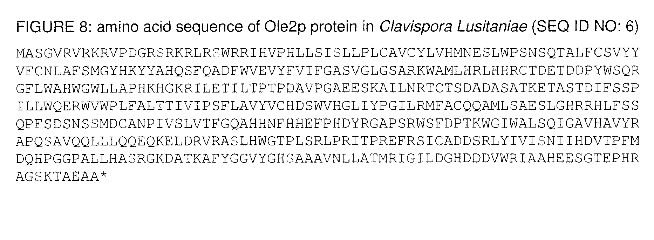

The strains as described above thus comprise a genetic modification of selected from a group comprising Δ6 desaturase, Δ9 desaturase, Δ12 desaturase, and/or Δ15 desaturase. Preferably, the isolated biologically pure hemiascomycetes yeast strains according to the present invention may further comprise a genetic modification of the *OLE2* gene which encodes a Δ9 desaturase. Most preferably, the *OLE2* gene is a partially or totally deleted. The nucleotide and amino acid sequences are are as set forth in Figures 7 and 8, respectively.

Strains that contain non functional desaturase genes have improved production of commodity chemicals of interest. Particularly, strains according to the present invention comprising a mutation of the *OLE2* gene are particularly useful in improved yield and production of lactones, such as decalactones when the yeasts are grown on suitable substrates like ricinoleic acid.

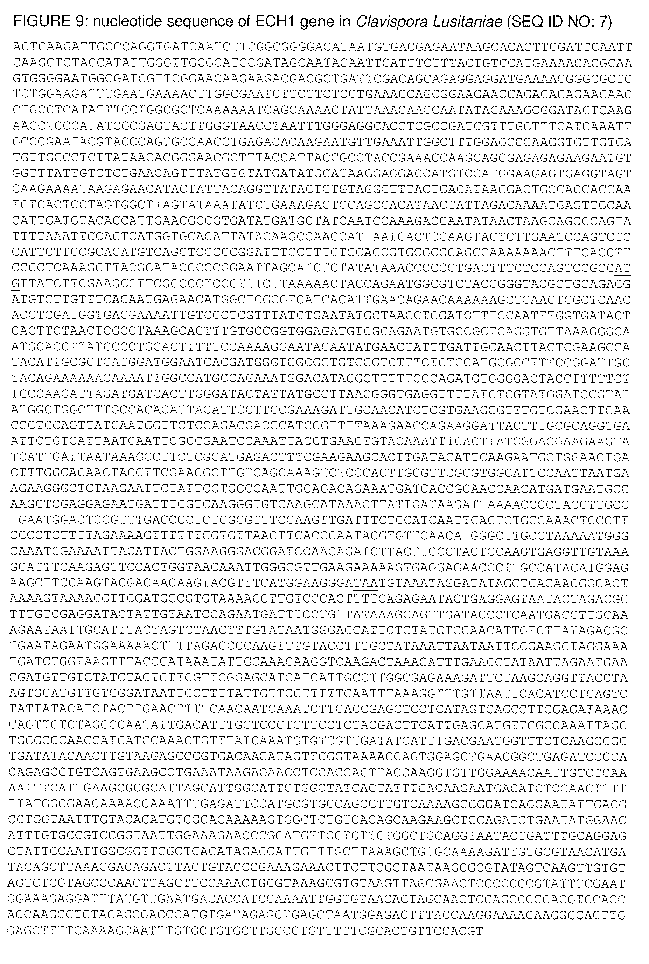

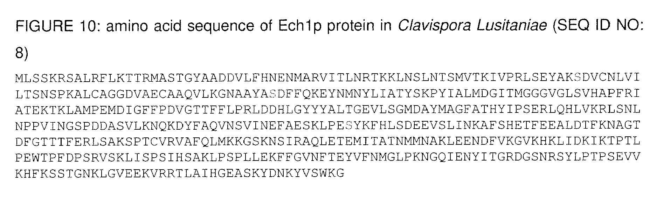

According to another aspect, the present invention relates to the orthologuous gene in *C. lusitaniae* of the human gene encoding a mitochondrial enoyl-coA hydratase. This novel gene has been designated *ECH1* (Enoyl-CoA Hydratase 1) by the Applicant. The identification and characterization of this novel gene confirms that certain hemiascomycetes yeast strains present a mitochondrial enzyme having an acetyl-coA deshydrogenase activity. Nucleotide and amino acid sequences of the novel *ECH1* gene are as set forth in Figures 9 and 10, respectively.

The isolated biologically pure hemiascomycetes yeast strains according to the present invention may thus comprise a genetic modification of the *ECH1* gene, preferably a partial or total deletion of this gene. These genetically modified strains result in an increase in the production of lactones like decalactone, when the strains are grown in a medium containing suitable substrates like ricinoleate.

According to the present invention, some of the preferred isolated biologically pure hemiascomycetes yeast mutant strains according are as follows:
*foX2*Δ;
*pxa1*Δ;
*fox2*Δ, *pxa 1*Δ;
*ole2*Δ;
*ole2*Δ, *fox2Δ*;
*ole2*Δ, *pxa 1*Δ;
*ole2*Δ, *fox2*Δ, *pxa1*Δ;
*ech 1*Δ;
*ech 1*Δ, *fox2*Δ;
*ech1*Δ, *pxa1*Δ;
*ech1*Δ, *fox2*Δ, *pxa1*Δ;
*ole2*Δ, *each1*Δ;
*ole2*Δ, *ech1*Δ, *fox2*Δ; *and*
*ole2*Δ, *ech1*Δ, *pxa1*Δ.

Furthermore, the Applicant has showed that the mutant yeasts were able to accumulate high amount of lipid compared to their dry weight, when grown under some specific conditions. The present invention thus relates to method of producing lipids and derived commodity materials using mutated yeasts for industrial purposes and method of optimizing such lipid production yield and rate.

The present invention further relates to a method of increasing the production of lipids and/or commodity materials as described above comprising the following steps:
a. providing the isolated strain as described above;
b. growing said strain in a suitable medium; and
c. recovering the lipids and/or commodity materials.

The method of the present invention hence facilitates the effective production of many commodity chemicals in high purity and yield including but not restricted to free fatty acids, long chain fatty acids, saturated, mono or polyunsaturated fatty acids (PUFAs), triacylglycerols (TAGs), phospholipids, phospholipids esters, phospholipids mono and/or diesters, biofuels/biodiesels, lipid derived fragrances and/or flavourings (bioflavours). The processes reported with the strains of the invention can be easily scaled up and hence by following the process of the present invention one can improve the working efficiency in industrial production.

The media and the fermentation conditions used for production of commodity chemicals is routine and well known in art. Such media may contain at least one carbon source which may be selected from a group comprising of castor oil, a castor oil hydrolysate, ricinoleic acid, palmitic acid, hydroxyl palmitic acid, stearic acid, hydroxyl stearic acid, oleic acid and derivatives, Massoi bark oil, lesquerolic acid etc... Preferably, the medium comprises carbon sources and is enriched in palmitic acid.

The media may also contain additional components like yeast extract, malt extract, polypeptone, and saccharides such as glucose. The media may contain a suitable nitrogen source which may be selected from a group comprising yeast extract, urea, corn steep liquor, ammonium sulfate, diammonium hydrogen phosphate, and the like. Chosen media may additionally contain cofactors selected from a group comprising inorganic salts such as manganese sulfate, calcium chloride, ferric chloride, ferrous sulfate, ferric sulfate, zinc sulfate, copper sulfate, magnesium sulfate, cobalt chloride, sodium molybdate, boron, and potassium iodide; coenzymes such as flavin mononucleotide (FMN), flavin adenine dinucleotide (FAD), nicotinamide adenine dinucleotide (NAD), nicotinamide adenine dinucleotide phosphate (NADP), and coenzyme A (CoA); nucleotides such as adenosine triphosphate (ATP); and vitamins such as L-carnitine, and vitamins of the group B.

A variety of fermentation process designs may be applied for commercial production of commodity chemicals using the strains as described above. For example, commercial production of commodity chemicals may be produced by a batch, fed-batch or continuous fermentation process.

A batch fermentation process is a closed system wherein the media composition is set at the beginning of the process and not subject to further additions beyond those required for maintenance of pH and oxygen level during the process. Thus, at the beginning of the culturing process the media is inoculated with the strains and growth or metabolic activity is permitted to occur without adding additional sources (*i*.*e*., carbon and nitrogen sources) to the medium. In batch processes the metabolite and biomass compositions of the system change constantly up to the time the culture is terminated. In a typical batch process, cells proceed through a static lag phase to a high-growth log phase and finally to a stationary phase, wherein the growth rate is diminished or halted. Left untreated, cells in the stationary phase will eventually die. A variation of the standard batch process is the fed-batch process, wherein the carbon source is continually added to the fermenter over the course of the fermentation process.

A fed-batch process is also suitable in the present invention. Fed-batch processes are useful when catabolite repression is apt to inhibit the metabolism of the cells or where it is desirable to have limited amounts of source in the media at any one time. Measurement of the carbon source concentration in fed-batch systems is difficult and therefore may be estimated on the basis of the changes of measurable factors such as pH, dissolved oxygen and the partial pressure of waste gases (e.g., CO₂). Batch and fed-batch culturing methods are common and well known in the art and examples may be found in Thomas D. Brock in Biotechnology: A Textbook of Industrial Microbiology, 2nd ed., (1989) Sinauer Associates: Sunderland, Mass.; or Deshpande, Mukund V., Appl. Biochem. Biotechnol., 36:227 (1992), herein incorporated by reference.

Commercial production of commodity chemicals using the strains according to the present invention may also be accomplished by a continuous fermentation process wherein a defined media is continuously added to a bioreactor while an equal amount of culture volume is removed simultaneously for product recovery. Continuous cultures generally maintain the cells in the log phase of growth at a constant cell density. Continuous or semi-continuous culture methods permit the modulation of one factor or any number of factors that affect cell growth or end product concentration. For example, one approach may limit the carbon source and allow all other parameters to moderate metabolism. In other systems, a number of factors affecting growth may be altered continuously while the cell concentration, measured by media turbidity, is kept constant. Continuous systems strive to maintain steady state growth and thus the cell growth rate must be balanced against cell loss due to media being drawn off the culture. Methods of modulating nutrients and growth factors for continuous culture processes, as well as techniques for maximizing the rate of product formation, are well known in the art of industrial microbiology.

In general, means for the purification of commodity chemicals may include extraction with organic solvents, sonication, supercritical fluid extraction (e.g., using carbon dioxide), saponification, fractional crystallization, HPLC, fractional distillation, silica gel chromatography, high-speed centrifugation or distillation and physical means such as presses, or combinations of these techniques. After extraction, the organic solvents can be removed by evaporation under a stream of nitrogen.

The following example describes in details the methods and techniques illustrative of the present invention.

### Examples

### Example 1

### 1.1 Strains and media

The *C. lusitaniae* strains constructed for this invention are listed in Table 1. All the mutant strains were derived from the wild-type strain 6936 *MAT***a** (Centraalbureau voor Schimmelcultures, Utrecht, The Nederlands). Yeasts were routinely cultivated in YPD (1% (w/v) yeast extract, 2% (w/v) peptone, 2% (w/v) glucose). Carbon assimilation tests were performed using YNB (0.17% (w/v) yeast nitrogen base without amino acids and without ammonium sulfate (Difco laboratories), 0.5% (w/v) ammonium sulfate) supplemented with 2% (w/v) of either glucose (so called YNB glucose) or other carbon sources. Counterselection of uracil auxotrophs after pop-out of the *URA3* marker gene in transformants was done on YNB glucose supplemented with 1 mg/ml of 5-fluoroorotic acid (5-FOA, Sigma Chemicals Co.) and 50 µg/ml uracil (1). For subcellular fractionation, the WOYglu medium made of YNB supplemented with 0.1% (w/v) yeast extract and 0.3% (w/v) glucose and the Induction medium containing 0.5% (w/v) bactopeptone, 0.3% (w/v) yeast extract, 0.12% (v/v) oleic acid, 0.2% (v/v) Tween 40 and 0.5% (w/v) KH₂PO₄ (adjusted to pH 6.0 with NaOH) was used. Yeasts were cultivated at 30°C, 35°C or 37°C, under constant agitation (230 rpm) for liquid cultures.

**Table 1 - Genotypes of the Candida lusitaniae strains constructed for this invention**

| **Strain** | **Genotype** | **Source or reference** |
|---|---|---|
| 6936 | *MAT***a** (wild-type) | CBS (Centraalbureau voor Schimmelcultures) |
| *ura3*[Δ360] | *MAT***a**, *ura3[Δ360]* | François *et al.,* 2004, El Kirat-Chatel et al., 2011 |
| *ura3*[Δ990] | *MAT***a**, *ura3*[Δ990] | El Kirat-Chatel et al., 2011 |
| *ic*/*1*Δ::GUN | *MAT***a**, *icl1*Δ::*URA3, ura3*Δ | This work |
| *icl1*Δ, *ura3*Δ | *MAT***a**, *icl1*Δ, *ura3*Δ | This work |
| *lcl1*Δ | *MAT***a**, *icl1*Δ, *URA3* | This work |
| *ICL1Re* | *MAT***a**, *ic*/*1*Δ::*ICL1-URA3*, *ura3*Δ | This work |
| *fox2*Δ::GUN | *MAT***a**, *fox2*Δ::*URA3*, *ura3*Δ | This work |
| *fox2*Δ, *ura3*Δ | *MAT***a**, *fox2*Δ, *ura3*Δ | This work |
| *fox2*Δ | *MAT***a**, *fox2*Δ, *URA3* | This work |
| *FOX2Re* | *MAT***a**, *fox2*Δ::*FOX2*-*URA3*, *ura3*Δ | This work |
| *ech1*Δ | *MAT***a**, *ech1*Δ, *URA3* | This work |
| *ole2*Δ::*URA3* | *MAT***a**, *ole2*Δ::*URA3*, *ura3*Δ | This work |
| *pxa1*Δ::*URA3* | *MAT***a**, *pxa1*Δ::*pGEMT*-*URA3*-*pxa1[core], ura3*Δ | This work This work |

### 1.2 DNA extractions

*C. lusitaniae* genomic DNA was extracted as described (4) from spheroplasts prepared using zymolyase (Euromedex). DNA concentrations were determined with a spectrophotometer at λ₂₆₀, using a conversion ratio of 1 OD₂₆₀ for 50 mg/ml DNA.

### 1.3 PCR amplifications

Hot-Star (Qiagen) or Pfu (Promega) Taq DNA polymerase was used for the PCR amplification of DNA fragments. PCR were performed as recommended by the supplier. All the primers were synthesized by Eurofins MWG Operon and are listed in Table 2.

**Table 2 - Oligonucleotides used in this work**

| **Primer** | **Sequence (5' to 3')^{1,2}** | Use |
|---|---|---|
| **NotURA3** | GACATGCAT**GCGGCCGC**ACAGAGGAGTAAGACAGG | Cloning *URA3* in complementation plasmids |
| **SpeURA3** | GCATCAC**ACTAGT**ACCCGTTGATGGCAGAGTTG | Cloning *URA3* in complementation plasmids |
| **BclGUN** | TCGAGA**TGATCA**CCCGAGGTCGCATGCTCC | Cloning *URA3* within *ICL1* ORF |
| **StuGUN** | CACGTG**AGGCCT**CCAAGCTATTTAGGTGACAC | Cloning *URA3* within *ICL1* ORF |
| **MfeGUN** | TCGAGA**CAATTG**CCCGAGGTCGCATGCTCC | Cloning *URA3* within *FOX2* ORF |
| **Bgl2GUN** | CGCGCG**AGATCT**CCAAGCTATTTAGGTGAC | Cloning *URA3* within *FOX2* ORF |
| **3'URA3F** | CTTCTCTATCCGTCTCTGTTCC | PCR characterization of transformants |
| **5'URA3R** | ACATCATCCTTCACCAACTCTGC | PCR characterization of transformants |
| **URA3del** | ACCGGTGACTCCATGAGCGTTG | PCR characterization of transformants |
| **URA3ex** | CTCAGCGTCAGGTGTTTACG | PCR characterization of transformants |
| **FICL1** | CGGTACATTACCTGTGCTTGC | Cloning *ICL1* in pGEM-T |
| **RICL1** | GAGCAGTTCAGGTAAATCCG | Cloning *ICL1* in pGEM-T |
| **FICL1ex** | ACTTCCAGATGGCTTCTCCA | PCR characterization of *icl1* transformants |
| **RICL1ex** | TTCAGTCCACATGGATGGTC | PCR characterization of *icl1* transformants |
| **FFOX2** | CGAGGTGTCACCATATAAGCC | Cloning *FOX2* in pGEM-T |
| **RFOX2** | GCTCACGAACGAAAGCCTAC | Cloning *FOX2* in pGEM-T |
| **FFOX2ex** | ATCTCGGTCCGTCATGAGTG | PCR characterization of *fox2* transformants |
| **RFOX2ex** | ATATGGCCTCCGAACAGAG | PCR characterization of *fox2* transformants |
| **5'Pxa1Ncol** | CAT**GCCATGGC**ACCAAGACGAAGGCGAG | Cloning PXACore in pG-URA3 |
| **3'Pxa1SacII** | TCA**CCGCGG**GCTCAACATATCGTCGTACG | Cloning PXACore in pG-URA3 |
| **5'Pxa1ex** | GACAGGTGCTATTGTTGGC | PCR characterization of *pxa* transformants |
| **3'Pxa1ex** | CGTATCCCCTTTGATCTCCAACAC | PCR characterization of *pxa* transformants |
| **5UpEch1** | CGCGAGTACTTGGGTAACCTA | *ECH1* deletion cassette |
| **3DoEch1** | TCTTACAAGTTGTATATCAGC | *ECH1* deletion cassette |
| **3UpGunEch1** | GACTGGAGAAAGTCAG aataaataatttgtgtttgccaaagcatttgaatggagaatgtcatAGGATA | *ECH1* deletion cassette |
| **5DoUraEch1** | TAGCTGAGAACGGCA | *ECH1* deletion cassette |
| **5Ech1nest** | CCCGAATACGTACCCAGTGCC | *ECH1* deletion cassette |
| **3Ech1nest** | GGATCATGGTTGGGCGCAGCT | *ECH1* deletion cassette |
| **FEch1ex** | GGAATGGCGATCGTTCGGAAC | *Cloning ECH1* |
| **REch1ex** | GACAAGGCTGGCACGCATGGA | *Cloning ECH1* |
| **exFEch1ex** | GGACATAATGTGACGAGAATAAGCACAC | PCR characterization of *ech1* transformants |
| **exREch1ex** | CAAATCAGTATTACCTGCAGCCAC TCGAACGTTTTACTTTTAGTGCCGTTCTCAGCTATATCC | PCR characterization of *ech1* transformants |
| **3UpEch1clean** | TGGCGGACTGGAGAAAGTCAG CATCTCTATATAAACCCCCCTGACTTTCTCCAGTCCGC | Ech1 cassette for URA3 pop-out |
| **5DoEch1clean** | CAGGATATAGCTGAGAACGGCA | Ech1 cassette for URA3 pop-out |
| **5Ech1cleanest** | GAAGACGACGCTGATTCGACAGCA | Ech1 cassette for URA3 pop-out |
| **3Ech1cleanest** | CTCAAATTTGGTTTTGTTCGCCATA | Ech1 cassette for URA3 pop-out |
| **5Gun** | GTGACACTATAGAATACTCAAGC | Forward URA3 cloned in pG-URA3 |
| **5URA** | CCCGTTGATGGCAGAGTTG | Forward URA3 |
| **3URA** | ATGACATTCTCCATTCAAATGC | Reverse URA3 |
| **5AmOLE2** | CCATAAATAAATGTCGCACAGC tatggaaaacatcatccttcaccaactctgccatcaacgggCGAACTCGA | *OLE2* Deletion cassette |
| **3AmOLE2URA3** | ACACCAGATGC taatttgtgtttgccaaagcatttgaatggagaatgtcatGAAGACAGCTGA | *OLE2* Deletion cassette |
| **5AvOLE2URA3** | AGCAGCG | *OLE2* Deletion cassette |
| **3AvOLE2** | GCGAGCACGCTGACTAAACC | *OLE2* Deletion cassette |

| | | |
|---|---|---|
| ¹ The sequences in bold characters correspond to a restriction endonuclease site whose name is included in the name of the primer *(Not*l*, Spel, Bcl*l*, Stu*l*, Mfel, Bglll, Sac*ll). ² Sequences in lower case letter are homologous to the *URA3* gene. | | |

### Example 2: Cloning of ICL1 and FOX2 genes of C. lusitaniae

BLAST analysis of the *C*. *lusitaniae* database (htt://www.broadinstitute.org/annotation/genome/candida group/) thereafter named BROAD Institute *Candida* genome database, allowed identification of a 1650-bp gene encoding a predicted protein of 549 amino acids (61 kDa) that bore a significant identity (79%) with isocitrate lyase of *C. albicans.* In the same way, we identified a 2709-bp gene encoding a predicted protein of 903 amino-acids (98 kDa) that bore significant identity (71%) with multifunctional-β-oxidation protein of *C. albicans.* The 1650-bp intronless *C*. *lusitaniae ICL1* gene was located on supercontig 2 from positions 409109 to 410755 (CLUG_01411.1) and the 2709-bp intronless *C. lusitaniae FOX2* gene was located on supercontig 2 from positions 281008 to 283713 (CLUG_01348.1). The complete *ICL1* and *FOX2* genes with their 5' and 3' non coding regions (300 and 500 bp for *ICL1* and 200 and 400 bp for *FOX2*) were isolated from the wild-type strain 6936 by PCR amplification and cloned into pGEM-T (Promega), to yield the plasmids pG-ICL1 and pG-FOX2.

### Example 3: Icl1Δ and fox2Δ mutant construction

*ICL1* and *FOX2* null mutants were constructed by using an improved integrative transformation system based upon the "*URA3*-blaster" strategy, adapted for *C*. *lusitaniae* by François *et al*.(1). The central part of the coding region of each cloned gene was deleted by digestion with adequate restriction enzymes from New England Biolabs (*Bcl*l and *Stu*l for *ICL1,* resulting in 701-bp deletion ; *Bgl*II and *Mfe*I for *FOX2*, resulting in 1543-bp deletion) and was replaced by the GUN fragment ; this fragment consisted of the *C*. *lusitaniae URA3* gene flanked on both sides by a noncoding 327-bp repeat (npt) obtained by amplification from the prokaryotic *NPTI* gene encoding neomycin phosphotransferase. The resulting disruption cassettes (Icl1Δ-GUN, Fox2A-GUN) were liberated from the plasmid backbone using Ndel and *Sph*I and were separately used to transform the strain *ura3_{[Δ360]}* to prototrophy, in order to obtain the mutant strains *icl1*Δ::GUN and *fox2*A::GUN. All the genotypes were confirmed by PCR and Southern Blot hybridization.

### Example 4: Complementation of icl1Δ and fox2A null mutant alleles

Selection of mutant strains that had excised the *URA3* marker was achieved by plating yeast cells from the mutant strains *icl1*Δ::GUN and *fox2*Δ::GUN onto YNB glucose plates containing 5FOA and uracil. Ura clones resistant to 5FOA were selected and their genetic organization, *i.e*. the loss of the *URA3* gene and of one of the flanking npt repetitive fragments, was confirmed by PCR and Southern Blot hybridization. The resulting mutant strains *icl1*Δ, *ura3*Δ and *fox2*Δ, *ura3*Δ were used further in two ways. First, they were transformed to uracil prototrophy by the complementation plasmid pG-URA3 to restore a functional *URA3* at its resident locus, allowing the selection of two new mutant strains having the genotypes *icl1*Δ, *ura3*_{[Δ360]}::*URA3*(abbreviated *icl1*Δ) and *fox2*Δ, *ura3*_{[Δ360]}:*:URA3* (abbreviated *fox2*Δ). This methodology was proved to be phenotypically neutral and avoids a possible position effect on the expression level of *URA3.*

Second, the strains *icl1*Δ, *ura3*Δ and *fox2*Δ, *ura3*Δ were transformed to uracil prototrophy by the complementation plasmids pG-ICL1-URA3 and pG-FOX2-URA3, obtained after insertion of an *URA3* allele at the *Not*l and *Spe*l restriction site of pG-ICL1 and pG-FOX2. Integration of the plasmids into the genome of the recipient strains was targeted at the *icl1*Δ and the *fox2*Δ loci, respectively, to give the "revertant" strains *ICL1Re* and *FOX2Re.* All genotypes were verified by PCR and Southern Blot hybridization; the genotype of the *fox2*Δ strain was also confirmed by nucleotide sequencing of the deleted locus.

### Example 5: Cloning of the PXA1 gene of C. lusitaniae and construction of the pxa1Δ mutant strain

BLAST analysis of the *C. lusitaniae* database (BROAD Institute *Candida* genome database) allowed identification of a 2430-bp gene encoding a predicted protein of 809 amino acids that bore a significant identity (66%) with the protein of *C. albicans.* The *C. lusitaniae PXA1* gene was located on supercontig 2 from positions 55051 to 57480 (CLUG_01238.1). The *pxa1*Δ mutant was constructed by disruption of the coding sequence of the wild allele. To do that, a 564-bp fragment corresponding to the core of the coding region of *PXA1* was amplified by PCR and cloned to the *Nco*l and *Sac*II restriction sites of the plasmid pG-URA3 (corresponding to a pGEM-T plasmid harbouring a cloned *URA3* gene). The resulting plasmid pG-URA3-pxa1 [core] was then linearized by *Afl*II*,* a single restriction site located within the *PXA1* cloned sequence, before transforming the strain *ura3*_{[Δ360]} to prototrophy. The *pxa1*Δ::*URA3* mutant strain was obtained after homologous integration of the plasmid at the *PXA1* locus.

### Example 6: Identification of the OLE1 and OLE2 gene of C. lusitaniae and construction of the ole2Δ mutant strain.

BLAST analysis of the *C. lusitaniae* database (BROAD Institute *Candida* genome database) allowed identification of two genes encoding proteins that bore a significant identity with the Ole1p and Ole2p proteins of *C. albicans.* The *C. lusitaniae OLE1* gene (CLUG_00176.1) was located on supercontig 1 from positions 345711 to 344260. The gene is intronless, has a size of 1452 bp, and encodes a predicted protein of 483 aa having 78.6% of identity with Ole1p of *C. albicans.* The *C. lusitaniae OLE2* gene (CLUG_05828.1) was located on supercontig 8 from positions 512253 to 513689. The gene is intronless, has a size of 1437 bp, and encodes a predicted protein of 478 aa having 45.2% of identity with Ole2p of *C*. *albicans.*

Several attempts to disrupt the *OLE1* gene of *C. lusitaniae* failed, which strongly suggested that *OLE1* was an essential gene for *C*. *lusitaniae,* as for *C*. *albicans.*

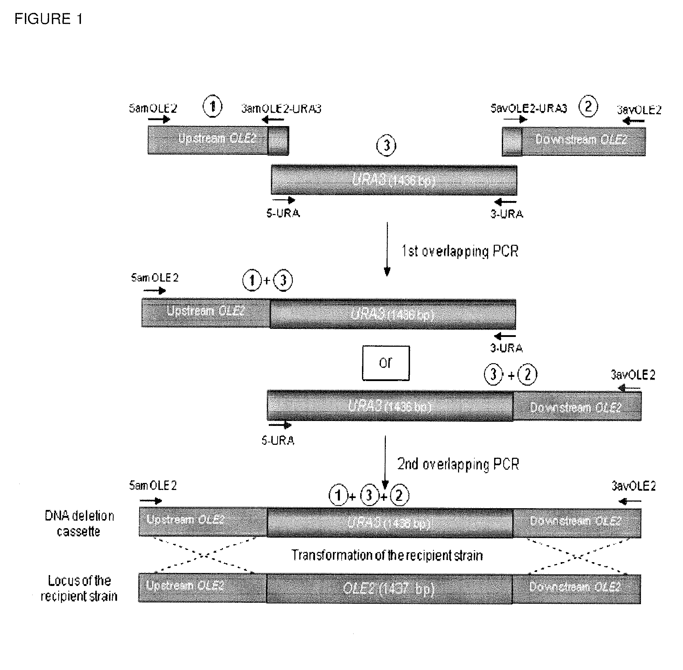

Deletion of *OLE2* was performed by homologous integration of a DNA cassette constructed by the cloning-free PCR-based method as previously described (21), using *URA3* as the marker gene of transformation. An *OLE2* deletion DNA cassette made of a 1400 bp fragment carrying the *URA3* gene, flanked by two 700 bp fragments corresponding to the upstream and downstream region of *OLE2,* was constructed by overlapping PCR (Fig. 1) using the specific oligonucleotides listed in Table 2. The *ole2*Δ::*URA3* mutant strain was obtained after transformation of the *ura3*Δ[990] strain to uracil prototophy by the homologous replacement of the wild *OLE2* locus with the *OLE2* deletion cassette.

### Example 7: Cloning the ECH1 gene of C. lusitaniae and construction of the ech1Δ mutant strain

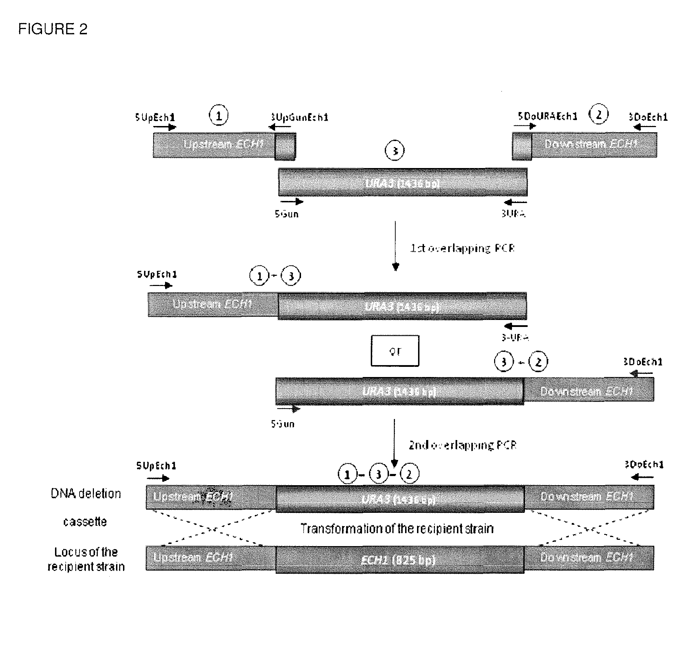

BLAST analysis of the *C. lusitaniae* database (BROAD Institute *Candida* genome database) allowed identification of a 1452-bp gene encoding a predicted protein of 483 amino acids that was homologous to the echAp mitochondrial enoyl-CoA hydratase of *Aspergillus nidulans* (30% identity, 46% similarity over partial alignment) and to the Ehd3p mitochondrial 3-hydroxyisobutyryl-CoA hydrolase (member of a family of enoyl-CoA hydratase/isomerases) of *Saccharomyces cerevisiae* (38.5% identity, 67.5% similarity). The *C. lusitaniae ECH1* gene was located on supercontig 4 from positions 1636544 to 1637992 (CLUG_04032.1). The *ech1*Δ::*URA3* mutant was obtained by homologous integration of a DNA cassette constructed by the cloning-free PCR-based method as previously described (21), according to the scheme of Fig. 2, and using the oligonucleotides listed in Table 2.

Additionally, the *URA3* marker of the *ech1*Δ::*URA3* mutant was excised by transforming the mutant strain with a cassette made of the upstream DNA fragment of *ECH1* fused to the downstream fragment of *ECH1*. Integration of the cassette by a double crossing-over at the *ech1*Δ locus led to the excision and loss of the *URA3* marker. Transformants that had excised *URA3* were selected onto YNB supplemented with 5FOA and uracil. The *URA3* wild allele was then reintroduced by homologous recombination at its own locus to give the final *ech1*Δ mutant.

### Example 8 Southern hybridizations

A least 2 µg of *C. lusitaniae* DNA was digested with *HindIII* or *EcoR*V and separated by electrophoresis in a 0.8% agarose gel. The DNA was transferred onto a nylon membrane (Hybond N⁺; Roche Molecular Biochemicals) and hybridized with digoxigenin-labeled DNA probes synthesized with a PCR DIG probe synthesis kit (Roche Molecular Biochemicals), as recommended by the supplier. *ICL1, FOX2, PXA1, ECH1, OLE2* and *URA3* DNA probes were generated by PCR amplification with the relevant primers (see Table 2). The *BLA* DNA probe, which was specific for the plasmidic ampicillin resistance gene, was also generated with specific primers.

### Example 9: DNA sequencing and sequence analysis

The nucleotide sequence of *FOX2, ICL1, PXA1, ECH1* and *OLE2* were determined, along with intergenic surrounding regions, and are provided in this study with their predicted translation products. Sequence reaction was realized using ABI Prism Dye Terminator Cycle Sequencing Ready Reaction^{®} v1.1 Kit (Applied Biosystems) from plasmidic DNA (300 à 600 ng) or PCR amplification (100 ng), as recommended by the supplier. Sequencing was done by the genotyping-sequencing facility of Bordeaux Segalen University. The similarity searches in databases were performed with the Basic Local Alignment Search Tool (BLAST) programs, available on the National Center for Biotechnology Information website (http://www.ncbi.nlm.nih.gov) and on the BROAD Institute website (http://www.broadinstitute.org/annotation/genome/candida_group/Blast.html). Consensus multiple alignments for nucleotide and amino acid sequences was done with the ClustaIW program, available on the European Bioinformatics Institute website (http://www.ebi.ac.uk/Tools/clustalw2/index.html).

### Example 10: In vitro growth assays

Growth assays were performed on solid YNB media containing 2% of either glucose, potassium acetate, ethanol, citrate, glycerol, oleic acid (C18:1), stearic acid (C18:0), palmitic acid (C16:0), myristic acid (C14:0), or capric acid (C10:0) as the sole carbon source. Inoculated media were incubated at room temperature, 30°C, or 37°C for 3 to 7 days, depending on the carbon source, as indicated in the figure legends. For the spot dilution assays, the strains were grown to mid-log phase in YPD, collected by centrifugation, washed with water, and resuspended in water to an OD₆₀₀ of 0.5. Cells were transferred to 96-well plates, and serially diluted fivefold. Drops of 7 µl of each dilution were deposited onto solid media. For growth assays in liquid media, strains were grown in YNB-glucose at 30°C or 37°C overnight. The next day, cells were harvested by centrifugation, washed twice with water, and resuspended at an OD₆₀₀ of 0.08 in YNB media containing the appropriate carbon source. Growth of the cultures was assessed over a period of 5 to 7 days by measuring the OD at 600 nm.

### Example 11: Purification of subcellular fractions with sucrose density gradient

Subcellular fractionation was adapted from Distel & Kragt protocol (3). Briefly, yeast strains were grown for 2 days in WOYglu at 35°C with agitation. When OD₆₀₀ reached 1.0, cells were harvested by centrifugation (4000 g, 5 min) and resuspended in 10 ml of induction medium. Then, 5 ml were used to inoculate one liter of induction medium. After 16 h of incubation at 30°C, yeasts were harvested, washed twice (4000 g, 5 min) with distilled water. Wet weight of the cell pellet was determined and cells were resuspended in 10 ml of buffer SH (β-mercapto-ethanol 0.5M, Tris-HCl 2.5 M, pH 9.3) per gram and incubated 10 minutes at 30°C under agitation. Then, cells were washed twice (4000 g, 5 min) in Tris-KCI buffer (KCI 0.5M, Tris 10 mM, pH 7). Yeasts were converted in spheroplasts after 1 hour of incubation at 30°C under agitation in 10 ml of Digestion Buffer (Sorbitol 1.35 M, EDTA 1 mM, Citrate phosphate 10 mM, pH 5.8) supplemented with 10 mg zymolyase per gram of fresh cells. All subsequent steps, including the separation of peroxisomes, were performed at 4°C. Spheroplasts were washed (4000 g, 10 min) twice in Spheroplast Buffer (Sorbitol 1.2 M in KEM (MES 5 mM, EDTA 1 mM, KCI 1 mM, pH 5.5)). Then, spheroplasts were broken by incubation 1 hour on ice in 10 ml homogenization buffer (Sorbitol 0.6 M in KEM, PMSF 1 mM) per gram of initial fresh cells. The homogenate was centrifuged at 1500 g for 10 min. The supernatant was saved, and the pellet was homogenized again in 20 ml of homogenization buffer. After 15 min on ice, the suspension was centrifuged; the supernatants were pooled and were further centrifuged at 20,000 g for 30 min. The organelle pellet was resuspended in 2 ml of sucrose 20% (w/w, in KEM).

Sucrose density gradient were adapted from Kamiryo *et al.* (9). The organelle fraction was applied onto a discontinuous gradient consisting of 4,5-, 4,5-, 9-, and 4,5- ml sucrose solutions of 25, 35, 42, and 53% (w/w in KEM), respectively. The tubes were centrifuged at 100,000 g for 1 h with an AH-629 swinging-bucket rotor (Sorvall). The small visible band at the 53 to 42% sucrose interphase contained the peroxysomal fraction, the larger one at the 42 to 35% interphase contained the mitochondrial fraction. Protein concentrations were estimated by the Bradford method, using bovine serum albumin as standard.

### Example 12: Assay of enzymes

Catalase (EC 1.11.1.6) activity was assayed using a Cary 100 scan (Varian) spectrophotometer at λ=240 nm and 20°C, as described by Aebi (11). The reaction mixture for catalase assay contained 10 µg protein sample, 50 mM potassium phosphate (pH 7) and 11 mM H₂0₂ was used to start the reaction. Cytochrome c oxidase (EC 1.9.3.1) activity was measured at λ 550 nm and 37 °C. Cytochrome c (Sigma) was previously reduced by equimolar addition of ascorbic acid. The reaction mixture contained 100 mM potassium phosphate (pH 6.9), 1 mM EDTA, and 28 µM cytochrome c reduced with ascorbic acid. The addition of protein sample was used to start the reaction.

### Example 13: Palmitoyl-CoA catabolism assay

Crude extracts (150 µg) or organelle fraction lysates (10 µg) were incubated at 37°C in 100 µL of reaction mixture containing 20 mM NAD, 10 mM CoASH, 1 mM FAD, 100 mM KCN, 2% Triton, 100 mM Tris-HCl pH 7.4. Reaction was started by the addition of 1µL of ¹⁴C_{α}-palmitoyl-CoA 10 µM (20 µCi/ml, Sigma-Aldrich). Reaction was stopped by the addition of 100 µL KOH 5M. After saponification (one hour at 65 °C), and acidification (addition of 100 µL H₂SO₄ 36 N), fatty acids were extracted with 2 mL of CHCl₃. Extracts were evaporated (under N₂), dissolved in 50 µL CHCl₃ / CH₃OH 2:1 (v/v) and the compounds were separated by thin layer chromatography (TLC) on 10x10 cm silica gel plates using 75:24:1 hexane/diethyl ether/acetic acid as solvent (v/v/v). Revelation was done using Phosphorimager Sl system (Amersham Biosciences). Analysis of ¹⁴C_{α}-palmitoyl-CoA consumption was then done using ImageQuant analysis software (Amersham Biosciences).

### Example 14: Lipid fatty acid composition

After growth on YPD or YNB + 2% palmitic acid, yeast were harvested and washed 3 times in water; amount of cells equivalent to 15 mg dry weight were then transformed into spheroplasts using zymolyase, as described above. To extract lipids from whole cells, two ml of chloroform/methanol (2:1, v/v) were added to the cell suspensions. After shaking and centrifugation, the organic phase was isolated and the remaining lipids were further extracted twice by the addition of 2 ml of chloroform to the aqueous phase and by shaking. The organic phases were then pooled and evaporated to dryness. Next, the lipids were redissolved in 70 µL of chloroform/methanol (2:1 v/v). Neutral lipids were purified from the extracts by one-dimensional TLC on silica gel plates (10x10 cm; Merck) using hexane /diethylether/acetic acid (90: 15: 2) as solvent. The lipids were then visualized by spraying the plates with a solution of 0.001% (w/v) primuline in 80% acetone, followed by exposure of the plates to UV light. The silica gel zones corresponding to the various lipids were then scraped from the plates and added to 1 mL of methanol / 2.5% H₂SO₄ containing 5 µg of heptadecanoic acid. After 1 h at 80°C, 1.5 mL of H₂O was added and fatty acid methyl esters (FAMES) were extracted with 0.75 mL of hexane. Separation of FAMES was performed by gas chromatography (GC) (Hewlett Packard 5890 series II; Hewlett-Packard, Palo Alto, CA, USA).

### Example 15: Lipid analysis by mass-spectrometry (MS)

Qualitative analyses were performed using an Agilent 6850 gas chromatograph equipped with an HP-5MS column (30 m x 0.25 mm x 0.25 µm) and an Agilent 5975 mass spectrometric detector (70 eV, mass-to-charge ratio 50-750). The initial temperature of 50°C was held for 1 min, increased at 50°C min⁻¹ to 200°C, held for 1 min at 200°C, increased again at 10°C min⁻¹ to 320°C, and held for 8 min at 320°C, with helium (1.5 mL min⁻¹) as carrier gas.

### Example 16: Mitochondrial respiration

Spheroplasts obtained as above were washed three times (2000 g, 10 min) in R buffer (sorbitol 1 M, EGTA 0.5 mM, MgSO₄ 2 mM, NaCl 1.7 mM, BSA 0.1 % and KH₂PO₄ 10 mM pH 7.2) and resuspended in the same buffer at a concentration of 125 mg/ml. Spheroplasts were then permeabilized with nystatin (40 µg/ml) during 10 minutes under gentle agitation. The respiratory activity was measured at 28°C with a Clark-type electrode connected to a computer giving an on-line display of rate values. Different substrates and inhibitors were added to the following final concentrations: NADH (4 mM), palmitoyl-CoA (0.2 mM), stearoyl-CoA (0.2 mM), valinomycin (5 µg/ml), myxothiazol (0.1 µg/ml), phenylsuccinate (5 mM). O₂ consumption rates are expressed in nmol O₂ per milligram of proteins and per milliliter (nmol O₂.mg⁻¹.ml⁻¹)

### Example 17: lmmunolocalization of Fox2p and Icl1p by western-blotting

Ten µg of proteins of the peroxisomal and mitochondrial fractions were separated by SDS-PAGE (sodium dodecyl sulfate-polyacrylamide gel electrophoresis), and transferred to polyvinylidene difluoride membrane. After a saturation phase in PBS-Tween-Milk (Phosphate 15 mM, pH 7.4, NaCl 154 mM, Tween 20 0.05% (v/v), milk powder 5%), the membrane was incubated with primary rabbit antiserum. The polyclonal antibody against Fox2p, designed against the *Candida tropicalis* multifunctional beta-oxidation enzyme, was graciously provided by W. Schliebs (Medizinisch Fakultät Ruhr-Universität Bochum, Germany). The polyclonal antibody against Icl1p, designed against the *Ashbya gossypii* isocitrate lyase, was a gift from S. Nieland (Hochschule Lausitz, Senftenberg, Germany). After 3 washing steps and incubation with a secondary anti-rabbit antibody coupled with peroxidase, immunodetection was realized in presence of 3,3'-diamino benzidine tetrahydrochloride and H₂O₂. The Quantity One software (BioRad) was used for the signal quantification.

## Claims

1. An isolated, biologically pure hemiascomycetes yeast strain comprising as metabolic pathways for the catabolism of fatty acid at least one peroxisomal β-oxidation *FOX2* independent pathway, at least one peroxisomal β-oxidation *FOX2* dependent pathway, and at least one mitochondrial β-oxidation *FOX2* dependent pathway.

2. An isolated, biologically pure hemiascomycetes yeast strain of claim 1 comprising as metabolic pathways for the catabolism of fatty acid (i) at least one *FOX2* independent peroxisomal β-oxidation pathway (ii) at least one *FOX2* dependent peroxisomal β-oxidation pathway, and (iii) one *FOX2* dependent mitochondrial β-oxidation pathway, wherein said strain still does grow on fatty acid carbon source when rendered non functional for the FOX2 gene.

3. The isolated strain according to claim 1 or 2, which is selected among the strains of the genus *Candida, Debaryomyces, Clavispora, Metschnikowia, Kluyveromyces, Lodderomyces, Pichia, Hansenula, Lipomyces, Yarrowia, and Geotrichum;* wherein strain of the genus *Candida* is preferably selected among *C. tropicalis, C. parapsilosis, C. orthopilosis, C. metapsilosis, C. norvegensis, C. antartica, C. dubliniensis, C. haemulonii, C. kefyr, C. krusei, C. lusitaniae, C. maltosa,* and *C. oleophila; and most preferably belongs to* the species *Candida lusitaniae* or *Clavispora lusitaniae;* wherein strain of the genus *Debaryomyces* is preferably selected among *D. hansenii* and *D. castelli.* Yeasts of the genus *Clavispora* include *C. lusitaniae and C. opuntiae;* wherein strain of the genus *Metschnikowia* is preferably *M. pulcherrima,* wherein strain of the genus *Kluyveromyces* is preferably selected among *K. lactis, K. marxianus, K. yarrowii,* and *K. wickerhamii,* wherein strain of the genus *Lodderomyces* is preferably *L. elongisporus;* wherein strain of the genus *Pichia* is preferably selected among *P. guilliermondii, P. anomala, P. fabianii, P. kluyveri, P. norvegensis, P. ohmeri, P. pastoris* and *P. angusta;* wherein strain of the genus *Hansenula* is preferably selected among *H. polymorpha;* wherein strain of the genus *Lipomyces* is preferably selected among *L. starkeyi, L. orientalis, L. yarrowii, and L. japonica;* wherein strain of the genus *Yarrowia* is preferably *Y. lipolytica;* wherein strain of the genus *Geotrichum* is preferably selected among *G. candidum, G. clavatum,* and *G*. *fici.*

4. The isolated strain according to any one of the preceding claims, wherein one or more pathways are disrupted or enhanced by at least one genetic modification, preferably by at least one or more genetic mutations, total or partial gene deletions or excisions, gene disruptions or integrations, and/or gene duplications.

5. The isolated strain according to claim 4, comprising one or more genetic modifications that disrupt or enhance the mitochondrial *FOX2* dependent pathway and/or the peroxisomal *FOX2* dependent pathway, preferably at least one more genetic modification of the *FOX2 gene,* such as preferably a total or partial gene deletion, excision, disruption insertion or integration within the *FOX2* gene, or most preferably wherein *FOX2* gene is rendered non functional by insertion of another gene of an unrelated metabolic pathway and/or by an antibiotic resistance gene (AB^{R}).

6. The isolated strain according to claim 5, wherein *FOX2* gene is enhanced and the strain comprises one or more duplication of the *FOX2* gene as set forth in SEQ ID NO: 1.

7. The isolate strain according to claim 5, wherein *FOX2* gene is rendered non functional by insertion of another gene of an unrelated metabolic pathway and/or by an antibiotic resistance gene (AB^{R}).

8. The isolated strain according to any one of preceding claims, further comprising a partial or total deletion or a genetic modification of the *PXA1* gene, or further comprising a genetic modification of the *ECH1* gene and/or of the *OLE2* gene.

9. The isolated strain according to any of the preceding claims, wherein said strain comprises genetic mutation and specific mutants are selected from a group comprising of
*a) fox2*Δ;
*b) pxa1*Δ;
*c) fox2*Δ, *pxa1*Δ;
*d) ole2*Δ;
*e) ole2*Δ, *fox2*Δ;
*f) ole2*Δ, *pxa1*Δ;
*g) ole2*Δ, *fox2*Δ, *pxa1*Δ;
*h) ech1*Δ;
*i) ech1*Δ *fox2*Δ;
*j) ech1*Δ, *pxa1*Δ;
*k) ech1*Δ, *fox2*Δ, *pxa1*Δ
*l) ole2*Δ, *each1*Δ;
*m) ole2*Δ, *ech1*Δ, *fox2*Δ; *and*
*n) ole2*Δ, *ech1*Δ, *pxa1*Δ.

10. Use of the isolated strain according to claim 5 or 9 for the production of long chain fatty acids, or for the production of triacylglycerols and ester derivatives, or for the production of phospholipids, phospholipids esters, phospholipids mono and/or di-esters, and biodiesel, or for the production of decalactone, or for the production of lipid derived flavorings and/or fragrances, or for the production of terpens, sesquiterpens, as well as adehydes and esters derivatives thereof.

11. Use according to claim 10, wherein said fatty acids are saturated, mono and/or polyunsaturated fatty acids, or wherein said fatty acids are in C₁₆-C₂₄, such as preferably linoleic acid or γ-linoleic acid.

12. Use of the isolated strain according to claim 6 for the production of lactones.

13. Use according to claim 10, wherein said lipid derived flavourings and/or fragrances are fruit and/or milk flavourings, preferably saturated and unsaturated δ- and γ-lactones, or wherein said lipid derived flavourings and/or fragrances are peach-like flavourings, preferably γ-decalactone, δ-decalactone, and/or γ-octalactone, or wherein said lipid derived flavorings and/or fragrances are coconut milk-like flavourings, preferably 4-dodecanolide and/or 4-octanolide.

14. A method of producing commodity chemicals comprising the following steps:
a. providing the isolated strain according to claim 5 or 9;
b. growing said strain in a suitable medium;
c. recovering said commodity chemicals.

15. The method of claim 14, wherein aid suitable medium comprises carbon source and is enriched one or more substrates chosen among palmitic acid, oleic acid, ricinoleic acid, or vegetable oil, and wherein said vegetable oil is preferably selected among sunflower oil, castor oil, coconut oil, Massoi bark oil, and/or hydrolysates thereof.


SEQUENCE LISTING

<110>  UNIVERSITE VICTOR SEGALEN BORDEAUX 2
       Centre Hospitalier Régional de Bordeaux
       CENTRE NATIONAL DE LA RECHERCHE SCIENTIFIQUE

<120>  NOVEL BETA-OXIDATION PATHWAY AND HEMICASCOMYCETES YEAST MUTANTS

<130>  1011-EPO

<160>  8

<170>  PatentIn version 3.3

<210>  1
<211>  3628
<212>  DNA
<213>  Clavispora lusitaniae

<400>  1
gcgtcgtaac aacatcgcat aattttttga gtttcgttcc tctttcaagg ttctctttat      60

ctcttcattt ctcctccttt actggtcagg attccacgga actaaatctt ctgtccgcta     120

cgtaacaatg ccccacacgg agcatgtgca aaccatgggt ggtcatgtgt gaagatgtac     180

gggtgtagtt tatccgagga tctcggtccg tcatgagtgt agtttttttc ggtactgggg     240

aaaaggtcga gcgtggcaat atgagcctgg caatactgcg cgccgattgt ctaccatgtg     300

gctgaaaaaa aaaaaactct accgaggtgt caccatataa gccatattgc aaaaacgttt     360

agccagacga aaccaaacag tttgtccgcg gagcaaacgc acccccctag attattcttg     420

gaagtctgtt tcttttctgg cgagagcgga gttcgtactt aagcgggttc cggcttcatt     480

gtgcacacct cggtatgtct ggggaaactt cacaagcgca ccccgcaaaa aaaaagatga     540

gtggatgtat aaaagggtat gacaaactcg aactaactcg cttctcttta gaaaccctag     600

ttttcacaaa ggttgagtta cactttattt atcttttcca ttatatatac tttcatcgat     660

gactgtctcc ttcaaggata aggttgtcgt tgtcaccgga gctggtggtg gcttaggtaa     720

attctactgt ttggagtttg cgaaaagagg cgccaaggtt gtggtaaatg acttgggtgg     780

ctctttgggc ggtcaaggcg gagattcaag ggccgctgat gttgtggtta atgagatcaa     840

gagtgccggc ggcattgctg tggctgatta caacaacgtg ttggatggcg aaaaaatcat     900

tgaaactgcc gttaaaaact ttggtactgt ccatgtcgtg gtcaacaatg ccggtatctt     960

gagagattct cagttcaaga agatgactga tgcagacttc cagttggttg ttgatgttca    1020

tgtcaatggt gctttcaaag tcaccaaagc cgcttgggaa tacttccgga aacaaaagta    1080

cggcagaatc ataaacaccg cctctccagc cggattgtat ggtaatttcg gccaggctaa    1140

ctattctgct gccaagatgg gtcttgttgg ttttgctgaa actttggcaa aagaaggcga    1200

gagatacaac atcaaggcta acactattgc tccattggcc agatctagaa tgactgaaaa    1260

ggtcttgccc cctccaattt tggagaagtt gaaccctgac aaggttgctc ctttggtgtt    1320

gtatttgtct tcagacgcca atgagagtat caacgggaaa atttttgaag ttgctgctgg    1380

tttctttgcc caaatccgct gggaacgttc agggggtgtc ttatttaagc cagatgactc    1440

ctttacaccg gaggctgtag caaagaagtt tgacgaaatc cgtgatttcg agagcagtgg 1500
taaaccagag ttgttgcagg ttcaacatcc agatcaactc aataactatt ttgagttgca 1560
aaaagccgcc agtcaattgg gacctaatga cactgctggt gttgatccag tgacattgaa 1620
tggaaaagtg gttttgatta ctggtgctgg ttccggcttg ggaagagact acgcgttggc 1680
ttttgctaag gctggcgcga aagttgttgt caatgacttt aaagatcctt ctcaagttgt 1740
cgaagagatt aagaaggctg gtggcgatgc tcacggtgac aaacatgatg ttgcttccca 1800
ggcgaaggag attattgata atgtcattgg caagtatggc acaatcgata ttttggtcaa 1860
taatgctgga attttgagag accgttcttt tgcaaaaatg tcctatgagg attggttaca 1920
agttcaaaag gtgcatttgg ctggcacgta ccagttgact agattggcgt ggccacattt 1980
ccttgaaaag aagtacggca gagttgtgaa tatcacttcg acctctggaa tctatggtaa 2040
ctttggccaa gccaattacg ccacggctaa agctgctatc gttggattga ccaggactct 2100
cgccattgaa ggttctagaa gtaacattaa ggttaatgtt gtggcgccac atgcggaaac 2160
ggctatgaca ttgaccgttt tcagagagga agataaaaac ttatatcctc cgaagttggt 2220
tgcgccattg ttaattttct tggcttctga gaatgtccct gtcactggtg agattttcga 2280
aggaggtgga ggttggattg gagccactag atggcaaaga gctaaaggtg ctgcttctaa 2340
agaagaacac attactcctg aatttgtccg tgaccacctt gctgacattg ttgacttctc 2400
tactgggact gcaaacccaa ctaccaccac tgaagcatct atggcgtttt tgagtgctgt 2460
tgaggatgat gacgatgatg atgaggacga agaggaagag gaagaagatg acgatgatga 2520
gaacccagac aagatgccag atcctgtgtt tacatggaac gaccgtgatg ttattttgta 2580
taatattgca gtcggagcca cccgtaagga gttgaagtat gtgtatgaga acgatctgga 2640
cttccaagtt gttccaacat ttacacactt gcctacattt aactctgtca agtctcagtt 2700
gactttttca aggttgttga gaaacttcaa tcctatgctt ttgttgcatg gcgaacacta 2760
catcaaaatc aacaagttcc caattccaat tgaggcatca gtgacaacgt cgtaccaacc 2820
catcacattg acccaaaaag gaactaatgc tattgtcgtt catggatcaa agtctgtcga 2880
cactgagact ggcgaggagc ttttctccaa cgaggctact cttttcatca gaaactgtga 2940
gtgtgacaac aaaaagtttg tggagcgcag atcgtttgct acaaactcct ttgctgcgcc 3000
aaagagtgag ccaactttct cccacgatat caagacttcg cctgaccaag ccgccttata 3060
cagattgaca ggcgacagaa atccattgca cattgatcct gcctttgcca aaggtgccaa 3120
atttgatgat cctattttgc atggtatgtg cacttatgga ttgagtgcta aggtattgtt 3180
ggatgagttt ggaccattca acgaaatcaa gggtagattc accggcattg ttttcccagg 3240
tgaaaccttg agggttcttg catggaagca aggtgatact gtgattttcc aaactcatgt 3300
ggtggacagg aagactatcg ctatcaacaa tgctgctatt aagttgattg gtgacaaggg 3360
caagttgtag ttttctagta catgacttat aatgaatggt ttattgagcg aaggtgagtg 3420
taagctgtat tcaatgtaaa ttgcatggct tgtcaagttt ataaagctac tggagacaga 3480

agttttcttt agtgaatttt tatgtctcag gagtgtgaaa tgtttctgtt tctgaaagta 3540

ggctttcttt cgtgagcgaa gtcgccagct aactctacag ggagcataaa agtcgtctat 3600

gtagctgaac aaagaacaca ttcaacag 3628

<210> 2
<211> 903
<212> PRT
<213> Clavispora lusitaniae

<400> 2

Met Thr Val Ser Phe Lys Asp Lys Val Val Val Val Thr Gly Ala Gly
1               5                   10                  15

Gly Gly Leu Gly Lys Phe Tyr Cys Leu Glu Phe Ala Lys Arg Gly Ala
            20                  25                  30

Lys Val Val Val Asn Asp Leu Gly Gly Ser Leu Gly Gly Gln Gly Gly
        35                  40                  45

Asp Ser Arg Ala Ala Asp Val Val Val Asn Glu Ile Lys Ser Ala Gly
    50                  55                  60

Gly Ile Ala Val Ala Asp Tyr Asn Asn Val Leu Asp Gly Glu Lys Ile
65                  70                  75                  80

Ile Glu Thr Ala Val Lys Asn Phe Gly Thr Val His Val Val Val Asn
                85                  90                  95

Asn Ala Gly Ile Leu Arg Asp Ser Gln Phe Lys Lys Met Thr Asp Ala
            100                 105                 110

Asp Phe Gln Leu Val Val Asp Val His Val Asn Gly Ala Phe Lys Val
        115                 120                 125

Thr Lys Ala Ala Trp Glu Tyr Phe Arg Lys Gln Lys Tyr Gly Arg Ile
    130                 135                 140

Ile Asn Thr Ala Ser Pro Ala Gly Leu Tyr Gly Asn Phe Gly Gln Ala
145                 150                 155                 160

Asn Tyr Ser Ala Ala Lys Met Gly Leu Val Gly Phe Ala Glu Thr Leu
                165                 170                 175

Ala Lys Glu Gly Glu Arg Tyr Asn Ile Lys Ala Asn Thr Ile Ala Pro
            180                 185                 190

Leu Ala Arg Ser Arg Met Thr Glu Lys Val Leu Pro Pro Pro Ile Leu
        195                 200                 205

Glu Lys Leu Asn Pro Asp Lys Val Ala Pro Leu Val Leu Tyr Leu Ser
    210                 215                 220

Ser Asp Ala Asn Glu Ser Ile Asn Gly Lys Ile Phe Glu Val Ala Ala
225                 230                 235                 240

Gly Phe Phe Ala Gln Ile Arg Trp Glu Arg Ser Gly Gly Val Leu Phe
                245                 250                 255

Lys Pro Asp Asp Ser Phe Thr Pro Glu Ala Val Ala Lys Lys Phe Asp
            260                 265                 270

Glu Ile Arg Asp Phe Glu Ser Ser Gly Lys Pro Glu Leu Leu Gln Val
        275                 280                 285

Gln His Pro Asp Gln Leu Asn Asn Tyr Phe Glu Leu Gln Lys Ala Ala
    290                 295                 300

Ser Gln Leu Gly Pro Asn Asp Thr Ala Gly Val Asp Pro Val Thr Leu
305                 310                 315                 320

Asn Gly Lys Val Val Leu Ile Thr Gly Ala Gly Ser Gly Leu Gly Arg
                325                 330                 335

Asp Tyr Ala Leu Ala Phe Ala Lys Ala Gly Ala Lys Val Val Val Asn
            340                 345                 350

Asp Phe Lys Asp Pro Ser Gln Val Val Glu Glu Ile Lys Lys Ala Gly
        355                 360                 365

Gly Asp Ala His Gly Asp Lys His Asp Val Ala Ser Gln Ala Lys Glu
    370                 375                 380

Ile Ile Asp Asn Val Ile Gly Lys Tyr Gly Thr Ile Asp Ile Leu Val
385                 390                 395                 400

Asn Asn Ala Gly Ile Leu Arg Asp Arg Ser Phe Ala Lys Met Ser Tyr
                405                 410                 415

Glu Asp Trp Leu Gln Val Gln Lys Val His Leu Ala Gly Thr Tyr Gln
            420                 425                 430

Leu Thr Arg Leu Ala Trp Pro His Phe Leu Glu Lys Lys Tyr Gly Arg
        435                 440                 445

Val Val Asn Ile Thr Ser Thr Ser Gly Ile Tyr Gly Asn Phe Gly Gln
    450                 455                 460

Ala Asn Tyr Ala Thr Ala Lys Ala Ala Ile Val Gly Leu Thr Arg Thr
465                 470                 475                 480

Leu Ala Ile Glu Gly Ser Arg Ser Asn Ile Lys Val Asn Val Val Ala
                485                 490                 495

Pro His Ala Glu Thr Ala Met Thr Leu Thr Val Phe Arg Glu Glu Asp
            500                 505                 510

Lys Asn Leu Tyr Pro Pro Lys Leu Val Ala Pro Leu Leu Ile Phe Leu
        515                 520                 525

Ala Ser Glu Asn Val Pro Val Thr Gly Glu Ile Phe Glu Gly Gly Gly
    530                 535                 540

Gly Trp Ile Gly Ala Thr Arg Trp Gln Arg Ala Lys Gly Ala Ala Ser
545                 550                 555                 560

Lys Glu Glu His Ile Thr Pro Glu Phe Val Arg Asp His Leu Ala Asp
                565                 570                 575

Ile Val Asp Phe Ser Thr Gly Thr Ala Asn Pro Thr Thr Thr Thr Glu
            580                 585                 590

Ala Ser Met Ala Phe Leu Ser Ala Val Glu Asp Asp Asp Asp Asp Asp
        595                 600                 605

Glu Asp Glu Glu Glu Glu Glu Glu Asp Asp Asp Asp Glu Asn Pro Asp
    610                 615                 620

Lys Met Pro Asp Pro Val Phe Thr Trp Asn Asp Arg Asp Val Ile Leu
625                 630                 635                 640

Tyr Asn Ile Ala Val Gly Ala Thr Arg Lys Glu Leu Lys Tyr Val Tyr
                645                 650                 655

Glu Asn Asp Ser Asp Phe Gln Val Val Pro Thr Phe Thr His Leu Pro
            660                 665                 670

Thr Phe Asn Ser Val Lys Ser Gln Leu Thr Phe Ser Arg Leu Leu Arg
        675                 680                 685

Asn Phe Asn Pro Met Leu Leu Leu His Gly Glu His Tyr Ile Lys Ile
    690                 695                 700

Asn Lys Phe Pro Ile Pro Ile Glu Ala Ser Val Thr Thr Ser Tyr Gln
705                 710                 715                 720

Pro Ile Thr Leu Thr Gln Lys Gly Thr Asn Ala Ile Val Val His Gly
                725                 730                 735

Ser Lys Ser Val Asp Thr Glu Thr Gly Glu Glu Leu Phe Ser Asn Glu
            740                 745                 750

Ala Thr Leu Phe Ile Arg Asn Cys Glu Cys Asp Asn Lys Lys Phe Val
        755                 760                 765

Glu Arg Arg Ser Phe Ala Thr Asn Ser Phe Ala Ala Pro Lys Ser Glu
    770                 775                 780

Pro Thr Phe Ser His Asp Ile Lys Thr Ser Pro Asp Gln Ala Ala Leu
785                 790                 795                 800

Tyr Arg Leu Thr Gly Asp Arg Asn Pro Leu His Ile Asp Pro Ala Phe
                805                 810                 815

Ala Lys Gly Ala Lys Phe Asp Asp Pro Ile Leu His Gly Met Cys Thr
            820                 825                 830

Tyr Gly Leu Ser Ala Lys Val Leu Leu Asp Glu Phe Gly Pro Phe Asn
        835                 840                 845

Glu Ile Lys Gly Arg Phe Thr Gly Ile Val Phe Pro Gly Glu Thr Leu
    850                 855                 860

Arg Val Leu Ala Trp Lys Gln Gly Asp Thr Val Ile Phe Gln Thr His
865                 870                 875                 880

Val Val Asp Arg Lys Thr Ile Ala Ile Asn Asn Ala Ala Ile Lys Leu
                885                 890                 895

Ile Gly Asp Lys Gly Lys Leu
            900

<210>  3
<211>  3281
<212>  DNA
<213>  Clavispora lusitaniae

<400>  3
gaactttgtt tctttgaaaa gaaaccattg cttggatttc ttgttttgct cttccttatt      60

aaccagtata taagaatgac tttttttttt tgattgacag ttgcggcacc tttatttctt     120

ctcgtgcacc tgattttttc cccctcttgt aaagtcgaaa aatcaaacct aaaatactca     180

aattcaccat aagcttttga tagttcatcc atttgatata cttctatatt atcttgcatt     240

ttcttaaatg gatactctca tgcttatagg ttattatttg ccatttaaat tatccggcac     300

accctcggaa gataatgtgg ggtagtattc gcgactacat ggtcattatg gctcattgct     360

agtctctctt cttaccgctg aaaaaccatg aatcgagtat caaagcttaa gctctataag     420

agagaagata ttcgaaagtt gctatctctt cttcgcgagt ttgtgctcgt atacaaagac     480

aaccaagtct ccatcaatta tcagagtcga ccggtcatcc tctttattgg tacggttgtg     540

tccacttttg gtatcggaac cttttttgct ctacgttcct tcattaactg gattagagat     600

ttgaaagata agaggctggc tcgtcgcccc cttctcatcc ggcaatcttc gactatacta     660

aaaaatggtt ctcgagagat ttacattccg aaaggaaaaa atggcacttc acggatcatt     720

attccaaagc caaatatgga tagatatgct gctgacaagt atctatacaa gaatttccat 780
atagatcaaa agttgaaact gcaaaataac ttgttcaact cgcgcttttt gaaccagcta 840
attatcattt ggagaatctt gatacctaaa ttcgcttcaa aaaacacata cttgctactt 900
tcgcaatgtt ttttcttggt tctccgcact tggctttctt tgatcattgc caaactcgat 960
ggccagattg ttaagaatat tattagtgca aacggcagaa aattcgtcag agatttaatt 1020
tattggattc tcattgcttt cccagcgtca tataccaatg cagccatcaa ataccttacg 1080
aatcgccttt cattgggctt tcggacaaac ttaacccggt acatccatga catgtacttg 1140
gataaagtaa tggcatatta caaaattctg ttgaattcga atgatattca gaatattgac 1200
caatacatca ctaatgatgt ggcaaaattt tgtgactcca tttgtggtct cttctcaagt 1260
atgggtaaac cattaattga cttggtattc ttctcagttt atttgagaga taacttgggg 1320
acaggtgcta ttgttggcat ttttgccaat tatttcgcta cagctgtcct cttgaaaaaa 1380
ttcacccctt cttttggcaa actttcatcc caaagaactc agttggaagg tcagtactat 1440
aacgaacatc tcaatctcat atctaactcc gaagaaatcg gtttttataa aggctcaaaa 1500
attgagaaag caaagttgcg agagactttc aatgagctta tgggccatgt ttcgaaggaa 1560
atcaatattt ccttctctta ttctattatt gaagattatg ttttgaagta cacgtggtcc 1620
gcttggggat acgtctttgc ttgtttgcca gtttttatgg aagaattctt ttcttccgag 1680
acaccccacc aagacgaagg cgagcttatt gaaccaacac ctaaaggtaa taaacctgtt 1740
gatgagcgcc agaacatgag gcagtttatc atcaacaaaa gattgatgtt gtctttagct 1800
gacgctggtt cgcggcttat gtactctatc aaagatgttt cggaacttac aggttacaca 1860
aaccgagtat ttatgttgtt gaccaactta catgtgagcc actcgccggt tttgcctttt 1920
ggatctaagt atggtgctgc agaccttaag ggaacagttc aacgaaacta cggtaacgga 1980
atccgctttg aaagcattcc cgtcattata cctagcactg aagggtctga aggtgttcta 2040
ttgatcgaca agttgaattt ccaagttcag gaacgtaaga accttttgat tttggggtcg 2100
aatggatgtg gaaaaacttc gatagcccgc attatggctg ggttatggcc tatttatcat 2160
ggtttgttat caaagcctag cgacgaggag atattttact tgcctcagaa aacgtatttc 2220
acaaatggaa atttgagaga ccaaatcatt tatccttttt cgtacgacga tatgttgggc 2280
atggggtaca atgatgatca tttgtatcac atattgaggg aagtaaaatt ggagtatctt 2340
ttaaccagag aaggaaactt caatgtgaag aaggattgga aggatgtatt cagcggtgga 2400
gaaaagcaaa gaatgagtgt cgctcgcgta ctctttgcta accccaaata tgtggttttg 2460
gatgaattca ctaacgcagt gagtactgat attgaagatt atctttttga acttcttcag 2520
agcaaaaaaa tcacattcat tacgctttca cataggccct tattgatgaa ataccatgac 2580
tacgtgttgg agatcaaagg ggatacgtct gaaaatcacg aatggctttt ccacgacttg 2640
acatcggagg agaacttgaa gtcgattgat aacgaaatca gcgatattca ggctaaattg 2700
gatcgtgtgc aggaatggga aaagcgaaaa catgacatcg aattatattt ggatggcaag 2760

tatgatgaaa ttcccgaaag cacgaatatg gtaatgactg cttcgtttga gctctaaaca 2820

gtccgtattg ggcgttgaat aatcaacttc atttcttcta taactattgt tttgccaata 2880

tatttttcat tatgtacact atattagttt tttcccttct cggccttcaa tgtaaaggca 2940

cacactcgcc gcccacccac gtgacgaacg aagcgtttct tttttaaacg atcgagcaaa 3000

ttatcctctg caacagatct tctttccctc atcttagaga aacaatgtcg gaaccaatca 3060

gaaaacacgg agatatttca aaagctccaa ctccccagaa cacaccatcc agcgtggcaa 3120

atctgtattt gaagcagcag cttcccagtg ttaacactat cacagaagaa gatgagaata 3180

acttgacgaa acagttggct aacaatccag cgttattgtc gatgattcaa ggtaaattgg 3240

gcactttggt aggccaacaa agtggatata tgaccaatta c 3281

<210> 4
<211> 809
<212> PRT
<213> Clavispora lusitaniae

<400> 4

Met Asn Arg Val Ser Lys Leu Lys Leu Tyr Lys Arg Glu Asp Ile Arg
1               5                   10                  15

Lys Leu Leu Ser Leu Leu Arg Glu Phe Val Leu Val Tyr Lys Asp Asn
            20                  25                  30

Gln Val Ser Ile Asn Tyr Gln Ser Arg Pro Val Ile Leu Phe Ile Gly
        35                  40                  45

Thr Val Val Ser Thr Phe Gly Ile Gly Thr Phe Phe Ala Leu Arg Ser
    50                  55                  60

Phe Ile Asn Trp Ile Arg Asp Leu Lys Asp Lys Arg Ser Ala Arg Arg
65                  70                  75                  80

Pro Leu Leu Ile Arg Gln Ser Ser Thr Ile Leu Lys Asn Gly Ser Arg
                85                  90                  95

Glu Ile Tyr Ile Pro Lys Gly Lys Asn Gly Thr Ser Arg Ile Ile Ile
            100                 105                 110

Pro Lys Pro Asn Met Asp Arg Tyr Ala Ala Asp Lys Tyr Leu Tyr Lys
        115                 120                 125

Asn Phe His Ile Asp Gln Lys Leu Lys Ser Gln Asn Asn Leu Phe Asn
    130                 135                 140

Ser Arg Phe Leu Asn Gln Leu Ile Ile Ile Trp Arg Ile Leu Ile Pro
145                 150                 155                 160

Lys Phe Ala Ser Lys Asn Thr Tyr Leu Leu Leu Ser Gln Cys Phe Phe
                165                 170                 175

Leu Val Leu Arg Thr Trp Leu Ser Leu Ile Ile Ala Lys Leu Asp Gly
            180                 185                 190

Gln Ile Val Lys Asn Ile Ile Ser Ala Asn Gly Arg Lys Phe Val Arg
        195                 200                 205

Asp Leu Ile Tyr Trp Ile Leu Ile Ala Phe Pro Ala Ser Tyr Thr Asn
    210                 215                 220

Ala Ala Ile Lys Tyr Leu Thr Asn Arg Leu Ser Leu Gly Phe Arg Thr
225                 230                 235                 240

Asn Leu Thr Arg Tyr Ile His Asp Met Tyr Leu Asp Lys Val Met Ala
                245                 250                 255

Tyr Tyr Lys Ile Ser Leu Asn Ser Asn Asp Ile Gln Asn Ile Asp Gln
            260                 265                 270

Tyr Ile Thr Asn Asp Val Ala Lys Phe Cys Asp Ser Ile Cys Gly Leu
        275                 280                 285

Phe Ser Ser Met Gly Lys Pro Leu Ile Asp Leu Val Phe Phe Ser Val
    290                 295                 300

Tyr Leu Arg Asp Asn Leu Gly Thr Gly Ala Ile Val Gly Ile Phe Ala
305                 310                 315                 320

Asn Tyr Phe Ala Thr Ala Val Leu Leu Lys Lys Phe Thr Pro Ser Phe
                325                 330                 335

Gly Lys Leu Ser Ser Gln Arg Thr Gln Leu Glu Gly Gln Tyr Tyr Asn
            340                 345                 350

Glu His Leu Asn Leu Ile Ser Asn Ser Glu Glu Ile Gly Phe Tyr Lys
        355                 360                 365

Gly Ser Lys Ile Glu Lys Ala Lys Leu Arg Glu Thr Phe Asn Glu Leu
    370                 375                 380

Met Gly His Val Ser Lys Glu Ile Asn Ile Ser Phe Ser Tyr Ser Ile
385                 390                 395                 400

Ile Glu Asp Tyr Val Leu Lys Tyr Thr Trp Ser Ala Trp Gly Tyr Val
                405                 410                 415

Phe Ala Cys Leu Pro Val Phe Met Glu Glu Phe Phe Ser Ser Glu Thr
            420                 425                 430

Pro His Gln Asp Glu Gly Glu Leu Ile Glu Pro Thr Pro Lys Gly Asn
        435                 440                 445

Lys Pro Val Asp Glu Arg Gln Asn Met Arg Gln Phe Ile Ile Asn Lys
    450                 455                 460

Arg Leu Met Leu Ser Leu Ala Asp Ala Gly Ser Arg Leu Met Tyr Ser
465                 470                 475                 480

Ile Lys Asp Val Ser Glu Leu Thr Gly Tyr Thr Asn Arg Val Phe Met
                485                 490                 495

Leu Leu Thr Asn Leu His Val Ser His Ser Pro Val Phe Ala Phe Gly
            500                 505                 510

Ser Lys Tyr Gly Ala Ala Asp Leu Lys Gly Thr Val Gln Arg Asn Tyr
        515                 520                 525

Gly Asn Gly Ile Arg Phe Glu Ser Ile Pro Val Ile Ile Pro Ser Thr
    530                 535                 540

Glu Gly Ser Glu Gly Val Leu Leu Ile Asp Lys Leu Asn Phe Gln Val
545                 550                 555                 560

Gln Glu Arg Lys Asn Leu Leu Ile Leu Gly Ser Asn Gly Cys Gly Lys
                565                 570                 575

Thr Ser Ile Ala Arg Ile Met Ala Gly Leu Trp Pro Ile Tyr His Gly
            580                 585                 590

Leu Leu Ser Lys Pro Ser Asp Glu Glu Ile Phe Tyr Leu Pro Gln Lys
        595                 600                 605

Thr Tyr Phe Thr Asn Gly Asn Leu Arg Asp Gln Ile Ile Tyr Pro Phe
    610                 615                 620

Ser Tyr Asp Asp Met Leu Gly Met Gly Tyr Asn Asp Asp His Leu Tyr
625                 630                 635                 640

His Ile Leu Arg Glu Val Lys Leu Glu Tyr Leu Leu Thr Arg Glu Gly
                645                 650                 655

Asn Phe Asn Val Lys Lys Asp Trp Lys Asp Val Phe Ser Gly Gly Glu
            660                 665                 670

Lys Gln Arg Met Ser Val Ala Arg Val Leu Phe Ala Asn Pro Lys Tyr
        675                 680                 685

Val Val Leu Asp Glu Phe Thr Asn Ala Val Ser Thr Asp Ile Glu Asp
    690                 695                 700

Tyr Leu Phe Glu Leu Leu Gln Ser Lys Lys Ile Thr Phe Ile Thr Leu
705                 710                 715                 720

Ser His Arg Pro Leu Leu Met Lys Tyr His Asp Tyr Val Leu Glu Ile
                725                 730                 735

Lys Gly Asp Thr Ser Glu Asn His Glu Trp Leu Phe His Asp Leu Thr
            740                 745                 750

Ser Glu Glu Asn Leu Lys Ser Ile Asp Asn Glu Ile Ser Asp Ile Gln
        755                 760                 765

Ala Lys Leu Asp Arg Val Gln Glu Trp Glu Lys Arg Lys His Asp Ile
    770                 775                 780

Glu Leu Tyr Leu Asp Gly Lys Tyr Asp Glu Ile Pro Glu Ser Thr Asn
785                 790                 795                 800

Met Val Met Thr Ala Ser Phe Glu Leu
                805

<210>  5
<211>  2105
<212>  DNA
<213>  Clavispora lusitaniae

<400>  5
gacgtaccgc tagaataaaa gattattcta cgtatgcact agcacccaat catacatgag      60

cttttctttc gtgtcttcat catcttgtaa ctaggaggca ccatatttgc agcctattta     120

cgaaggcatg catgatcgaa ataatgcaat aaacgcaatg cacacccctt cttgccgttt     180

tagttgtatc ttgtggttgt aaagatctca aacataagcg gggtagctcc agaatactat     240

agaaagttgt cctttttccg ctttgccacg taaaatatca aaacggtcca tatgcatcaa     300

cgcacataat caattggata acgaggccag taattgccgc tctatttacc agcagaagca     360

agtagctttt atcacataaa tgggtgattt atctatttct atgaattttt ttctactttg     420

tatatcctgt ttgcagcaat atcgtttatg ctcgtttatt ctgacgtggt aatcatctgc     480

gtgcaagaat cacgtgcatc acatccccac taaccactct cattaaaagc ttcttgattg     540

ccttcacatc tacgctactt cttcgttcag tttccattct tccttttttt ttgcatatgg     600

catctggtgt tcgagttcga aagcgggtgc ctgatgggcg cctgcggaag agacttcgtc     660

tgtggcgccg gatccatgtt ccacaccttc tttcgatcct gctacttccc ctttgcgctg     720

tgtgctactt agtgcacatg aacgagagtt tgtggccttc taattcccaa acagccttgt     780

tctgttctgt ctactacgta ttctgcaatt tggcattctc catgggctac cataagtact     840

atgcgcatca aagtttccaa gcagattttt gggtcgaggt gtactttgtg atttttggag     900

ctagtgtagg gcttggttct gcccgaaaat gggctatgtt gcaccgttta catcacaggt     960

gtactgacga gacggatgat ccgtattgga gccaacgtgg attcttatgg gcacattggg    1020

gttggctatt agcaccgcac aagcatggta agagaatctt ggaaactatt ttgaccccaa    1080

cacccgacgc tgtgcctgga gctgaagaaa gcaaagcaat acttaatcgc acttgcacct 1140

cggatgctga tgcatctgcc actaaagaga ctgctagtac tgatatcttc tcctcaccaa 1200

ttctactatg gcaagagcgc tgggtatggc cactatttgc tctcaccacg atagtgattc 1260

catcttttct tgctgtctat gtttgccacg actcttgggt gcacggtctt atatatccag 1320

gcattctccg catgtttgct tgtcagcaag ctatgctttc agcagaatca ttgggccaca 1380

ggcgacattt attttcctcg caacctttca gtgactctaa ctcgctgatg gactgtgcta 1440

atcctatagt ttccctagtc acttttggtc aggcacatca caatttccat catgagttcc 1500

ctcatgatta tcgtggagca ccatctagat ggtcatttga tccaaccaaa tgggggatct 1560

gggccttgtc ccaaattggt gcggtacatg ccgtatatcg ggccccgcaa ctggctgtgc 1620

aacagctact tctccaacag gaacaaaagg aactagatcg tgtccgtgcg ctgctacatt 1680

ggggtacacc gctttcacgt ctaccgcgta tcactcctcg ggagtttcgc agcatttgtg 1740

ccgacgattc acgcctttac atcgtaattc tgaacattat acacgatgtg acgcctttta 1800

tggatcagca tcctggtggg ccggctctat tacatgccct gcgtggtaag gatgctacaa 1860

aagcgttcta tggaggcgtg tacggacatc tggctgcagc agtgaatttg ctcgcaacaa 1920

tgcgtattgg cattcttgac ggccatgatg atgatgtatg gaggatcgcc gcacacgagg 1980

aaagtgggac tgagccacat cgtgctggcc tgaagacagc tgaagcagcg tgaagcacct 2040

tcggcacatg atgacaccta tcgaaattat ttcgtcaaat gatcttggcg tacttggtat 2100

ataca 2105

<210> 6
<211> 478
<212> PRT
<213> Clavispora lusitaniae

<400> 6

Met Ala Ser Gly Val Arg Val Arg Lys Arg Val Pro Asp Gly Arg Ser
1               5                   10                  15

Arg Lys Arg Leu Arg Ser Trp Arg Arg Ile His Val Pro His Leu Leu
            20                  25                  30

Ser Ile Ser Leu Leu Pro Leu Cys Ala Val Cys Tyr Leu Val His Met
        35                  40                  45

Asn Glu Ser Leu Trp Pro Ser Asn Ser Gln Thr Ala Leu Phe Cys Ser
    50                  55                  60

Val Tyr Tyr Val Phe Cys Asn Leu Ala Phe Ser Met Gly Tyr His Lys
65                  70                  75                  80

Tyr Tyr Ala His Gln Ser Phe Gln Ala Asp Phe Trp Val Glu Val Tyr
                85                  90                  95

Phe Val Ile Phe Gly Ala Ser Val Gly Leu Gly Ser Ala Arg Lys Trp
            100                 105                 110

Ala Met Leu His Arg Leu His His Arg Cys Thr Asp Glu Thr Asp Asp
        115                 120                 125

Pro Tyr Trp Ser Gln Arg Gly Phe Leu Trp Ala His Trp Gly Trp Leu
    130                 135                 140

Leu Ala Pro His Lys His Gly Lys Arg Ile Leu Glu Thr Ile Leu Thr
145                 150                 155                 160

Pro Thr Pro Asp Ala Val Pro Gly Ala Glu Glu Ser Lys Ala Ile Leu
                165                 170                 175

Asn Arg Thr Cys Thr Ser Asp Ala Asp Ala Ser Ala Thr Lys Glu Thr
            180                 185                 190

Ala Ser Thr Asp Ile Phe Ser Ser Pro Ile Leu Leu Trp Gln Glu Arg
        195                 200                 205

Trp Val Trp Pro Leu Phe Ala Leu Thr Thr Ile Val Ile Pro Ser Phe
    210                 215                 220

Leu Ala Val Tyr Val Cys His Asp Ser Trp Val His Gly Leu Ile Tyr
225                 230                 235                 240

Pro Gly Ile Leu Arg Met Phe Ala Cys Gln Gln Ala Met Leu Ser Ala
                245                 250                 255

Glu Ser Leu Gly His Arg Arg His Leu Phe Ser Ser Gln Pro Phe Ser
            260                 265                 270

Asp Ser Asn Ser Ser Met Asp Cys Ala Asn Pro Ile Val Ser Leu Val
        275                 280                 285

Thr Phe Gly Gln Ala His His Asn Phe His His Glu Phe Pro His Asp
    290                 295                 300

Tyr Arg Gly Ala Pro Ser Arg Trp Ser Phe Asp Pro Thr Lys Trp Gly
305                 310                 315                 320

Ile Trp Ala Leu Ser Gln Ile Gly Ala Val His Ala Val Tyr Arg Ala
                325                 330                 335

Pro Gln Ser Ala Val Gln Gln Leu Leu Leu Gln Gln Glu Gln Lys Glu
            340                 345                 350

Leu Asp Arg Val Arg Ala Ser Leu His Trp Gly Thr Pro Leu Ser Arg
        355                 360                 365

Leu Pro Arg Ile Thr Pro Arg Glu Phe Arg Ser Ile Cys Ala Asp Asp
    370                 375                 380

Ser Arg Leu Tyr Ile Val Ile Ser Asn Ile Ile His Asp Val Thr Pro
385                 390                 395                 400

Phe Met Asp Gln His Pro Gly Gly Pro Ala Leu Leu His Ala Ser Arg
                405                 410                 415

Gly Lys Asp Ala Thr Lys Ala Phe Tyr Gly Gly Val Tyr Gly His Ser
            420                 425                 430

Ala Ala Ala Val Asn Leu Leu Ala Thr Met Arg Ile Gly Ile Leu Asp
        435                 440                 445

Gly His Asp Asp Asp Val Trp Arg Ile Ala Ala His Glu Glu Ser Gly
    450                 455                 460

Thr Glu Pro His Arg Ala Gly Ser Lys Thr Ala Glu Ala Ala
465                 470                 475

<210>  7
<211>  4201
<212>  DNA
<213>  Clavispora lusitaniae

<400>  7
actcaagatt gcccaggtga tcaatcttcg gcggggacat aatgtgacga gaataagcac      60

acttcgattc aattcaagct ctaccatatt gggttgcgca tccgatagca atacaattca     120

tttctttact gtccatgaaa acacgcaagt ggggaatggc gatcgttcgg aacaagaaga     180

cgacgctgat tcgacagcag aggaggatga aaacgggcgc tctctggaag atttgaatga     240

aaacttggcg aatcttcttc tcctgaaacc agcggaagaa cgagagagag aagaacctgc     300

ctcatatttc ctggcgctca aaaaatcagc aaaactatta aacaaccaat atacaaagcg     360

gatagtcaag aagctcccat atcgcgagta cttgggtaac ctaatttggg aggcacctcg     420

ccgatcgttt gctttcatca aattgcccga atacgtaccc agtgccaacc tgagacacaa     480

gaatgttgaa attggctttg gagcccaagg tgttgtgatg ttggcctctt ataacacggg     540

aacgctttac cattaccgcc taccgaaacc aagcagcgag agagaagaat gtggtttatt     600

gtctctgaac agtttatgtg tatgatatgc ataaggagga gcatgtccat ggaagagtga     660

ggtagtcaag aaaataagag aacatactat tacaggttat actctgtagg ctttactgac     720

ataaggactg ccaccaccaa tgtcactcct agtggcttag tataaatatc tgaaagactc     780

cagccacata actattagac aaaatgagtt gcaacattga tgtacagcat tgaacgccgt     840

gatatgatgc tatcaatcca aagaccaata taactaagca gcccagtatt ttaaattcca     900

ctcatggtgc acattataca agccaagcat taatgactcg aagtactctt gaatccagtc     960

tccattcttc cgcacatgtc agctcccccg gatttccttt ctccagcgtg cgcgcagcca    1020

aaaaaacttt caccttcccc tcaaaggtta cgcatacccc cggaattagc atctctatat 1080
aaacccccct gactttctcc agtccgccat gttatcttcg aagcgttcgg ccctccgttt 1140
cttaaaaact accagaatgg cgtctaccgg gtacgctgca gacgatgtct tgtttcacaa 1200
tgagaacatg gctcgcgtca tcacattgaa cagaacaaaa aagctcaact cgctcaacac 1260
ctcgatggtg acgaaaattg tccctcgttt atctgaatat gctaagctgg atgtttgcaa 1320
tttggtgata ctcacttcta actcgcctaa agcactttgt gccggtggag atgtcgcaga 1380
atgtgccgct caggtgttaa agggcaatgc agcttatgcc ctggactttt tccaaaagga 1440
atacaatatg aactatttga ttgcaactta ctcgaagcca tacattgcgc tcatggatgg 1500
aatcacgatg ggtggcggtg tcggtctttc tgtccatgcg cctttccgga ttgctacaga 1560
aaaaacaaaa ttggccatgc cagaaatgga cataggcttt ttcccagatg tggggactac 1620
ctttttcttg ccaagattag atgatcactt gggatactat tatgccttaa cgggtgaggt 1680
tttatctggt atggatgcgt atatggctgg ctttgccaca cattacattc cttccgaaag 1740
attgcaacat ctcgtgaagc gtttgtcgaa cttgaaccct ccagttatca atggttctcc 1800
agacgacgca tcggttttaa agaaccagaa ggattacttt gcgcaggtga attctgtgat 1860
taatgaattc gccgaatcca aattacctga actgtacaaa tttcacttat cggacgaaga 1920
agtatcattg attaataaag ccttctcgca tgagactttc gaagaagcac ttgatacatt 1980
caagaatgct ggaactgact ttggcacaac taccttcgaa cgcttgtcag caaagtctcc 2040
cacttgcgtt cgcgtggcat tccaattaat gaagaagggc tctaagaatt ctattcgtgc 2100
ccaattggag acagaaatga tcaccgcaac caacatgatg aatgccaagc tcgaggagaa 2160
tgatttcgtc aagggtgtca agcataaact tattgataag attaaaaccc ctaccttgcc 2220
tgaatggact ccgtttgacc cctctcgcgt ttccaagttg atttctccat caattcactc 2280
tgcgaaactc ccttcccctc ttttagaaaa gttttttggt gttaacttca ccgaatacgt 2340
gttcaacatg ggcttgccta aaaatgggca aatcgaaaat tacattactg gaagggacgg 2400
atccaacaga tcttacttgc ctactccaag tgaggttgta aagcatttca agagttccac 2460
tggtaacaaa ttgggcgttg aagaaaaagt gaggagaacc cttgccatac atggagaagc 2520
ttccaagtac gacaacaagt acgtttcatg gaagggataa tgtaaatagg atatagctga 2580
gaacggcact aaaagtaaaa cgttcgatgg cgtgtaaaag gttgtcccac ttttcagaga 2640
atactgagga gtaatactag acgctttgtc gaggatacta ttgtaatcca gaatgatttc 2700
ctgttataaa gcagttgata ccctcaatga cgttgcaaag aataattgca tttactagtc 2760
taactttgta taatgggacc attctctatg tcgaacattg tcttatagac gctgaataga 2820
atggaaaaac ttttagaccc caagtttgta cctttgctat aaattaataa ttccgaaggt 2880
aggaaatgat ctggtaagtt taccgataaa tattgcaaag aaggtcaaga ctaaacattt 2940
gaacctataa ttagaatgaa cgatgttgtc tatctactct tcgttcggag catcatcatt 3000
gccttggcga gaaagattct aagcaggtta cctaagtgca tgttgtcgga taattgcttt 3060

tattgttggt ttttcaattt aaaggtttgt taattcacat cctcagtcta ttatacatct 3120

acttgaactt ttcaacaatc aaatcttcac cgagctcctc atagtcagcc ttggagataa 3180

accagttgtc tagggcaata ttgacatttg ctccctcttc ctctacgact tcattgagca 3240

tgttcgccaa attagctgcg cccaaccatg atccaaactg tttatcaaat gtgtcgttga 3300

tatcatttga cgaatggttt ctcaaggggc tgatatacaa cttgtaagag ccggtgacaa 3360

gatagttcgg taaaaccagt ggagctgaac ggctgagatc cccacagagc ctgtcagtga 3420

agcctgaaat aagagaacct ccaccagtta ccaaggtgtt ggaaaacaat tgtctcaaaa 3480

tttcattgaa gcgcgcatta gcattggcat tctggctatc actatttgac aagaatgaca 3540

tctccaagtt ttttatggcg aacaaaacca aatttgagat tccatgcgtg ccagccttgt 3600

caaaagccgg atcaggaata ttgacgcctg gtaatttgta cacatgtggc acaaaaagtg 3660

gctctgtcac agcaagaagc tccagatctg aatatggaac atttgtgccg tccggtaatt 3720

ggaaagaacc cggatgttgg tgttgtggct gcaggtaata ctgatttgca ggagctattc 3780

caattggcgg ttcgctcaca tagagcattg tttgcttaaa gctgtgcaaa agattgtgcg 3840

taacatgata cagcttaaac gacagactta ctgtacccga aagaaacttc ttcggtaata 3900

agcgcgtata gtcaagttgt gtagtctcgt agcccaactt agcttccaaa ctgcgtaaag 3960

cgtgtaagtt agcgaagtcg cccgcgtatt tcgaatggaa agaggattta tgttgaatga 4020

caccatccaa aattggtgta acactagcaa ctccagcccc cacgtccacc accaagcctg 4080

tagagcgacc catgtgatag agctgagcta atggagactt taccaaggaa aacaagggca 4140

cttggaggtt ttcaaaagca atttgtgctg tgcttgccct gtttttcgca ctgttccacg 4200

t 4201

<210> 8
<211> 483
<212> PRT
<213> Clavispora lusitaniae

<400> 8

Met Leu Ser Ser Lys Arg Ser Ala Leu Arg Phe Leu Lys Thr Thr Arg
1               5                   10                  15

Met Ala Ser Thr Gly Tyr Ala Ala Asp Asp Val Leu Phe His Asn Glu
            20                  25                  30

Asn Met Ala Arg Val Ile Thr Leu Asn Arg Thr Lys Lys Leu Asn Ser
        35                  40                  45

Leu Asn Thr Ser Met Val Thr Lys Ile Val Pro Arg Leu Ser Glu Tyr
    50                  55                  60

Ala Lys Ser Asp Val Cys Asn Leu Val Ile Leu Thr Ser Asn Ser Pro
65                  70                  75                  80

Lys Ala Leu Cys Ala Gly Gly Asp Val Ala Glu Cys Ala Ala Gln Val
                85                  90                  95

Leu Lys Gly Asn Ala Ala Tyr Ala Ser Asp Phe Phe Gln Lys Glu Tyr
            100                 105                 110

Asn Met Asn Tyr Leu Ile Ala Thr Tyr Ser Lys Pro Tyr Ile Ala Leu
        115                 120                 125

Met Asp Gly Ile Thr Met Gly Gly Gly Val Gly Leu Ser Val His Ala
    130                 135                 140

Pro Phe Arg Ile Ala Thr Glu Lys Thr Lys Leu Ala Met Pro Glu Met
145                 150                 155                 160

Asp Ile Gly Phe Phe Pro Asp Val Gly Thr Thr Phe Phe Leu Pro Arg
                165                 170                 175

Leu Asp Asp His Leu Gly Tyr Tyr Tyr Ala Leu Thr Gly Glu Val Leu
            180                 185                 190

Ser Gly Met Asp Ala Tyr Met Ala Gly Phe Ala Thr His Tyr Ile Pro
        195                 200                 205

Ser Glu Arg Leu Gln His Leu Val Lys Arg Leu Ser Asn Leu Asn Pro
    210                 215                 220

Pro Val Ile Asn Gly Ser Pro Asp Asp Ala Ser Val Leu Lys Asn Gln
225                 230                 235                 240

Lys Asp Tyr Phe Ala Gln Val Asn Ser Val Ile Asn Glu Phe Ala Glu
                245                 250                 255

Ser Lys Leu Pro Glu Ser Tyr Lys Phe His Leu Ser Asp Glu Glu Val
            260                 265                 270

Ser Leu Ile Asn Lys Ala Phe Ser His Glu Thr Phe Glu Glu Ala Leu
        275                 280                 285

Asp Thr Phe Lys Asn Ala Gly Thr Asp Phe Gly Thr Thr Thr Phe Glu
    290                 295                 300

Arg Leu Ser Ala Lys Ser Pro Thr Cys Val Arg Val Ala Phe Gln Leu
305                 310                 315                 320

Met Lys Lys Gly Ser Lys Asn Ser Ile Arg Ala Gln Leu Glu Thr Glu
                325                 330                 335

Met Ile Thr Ala Thr Asn Met Met Asn Ala Lys Leu Glu Glu Asn Asp
            340                 345                 350

Phe Val Lys Gly Val Lys His Lys Leu Ile Asp Lys Ile Lys Thr Pro
        355                 360                 365

Thr Leu Pro Glu Trp Thr Pro Phe Asp Pro Ser Arg Val Ser Lys Leu
    370                 375                 380

Ile Ser Pro Ser Ile His Ser Ala Lys Leu Pro Ser Pro Leu Leu Glu
385                 390                 395                 400

Lys Phe Phe Gly Val Asn Phe Thr Glu Tyr Val Phe Asn Met Gly Leu
                405                 410                 415

Pro Lys Asn Gly Gln Ile Glu Asn Tyr Ile Thr Gly Arg Asp Gly Ser
            420                 425                 430

Asn Arg Ser Tyr Leu Pro Thr Pro Ser Glu Val Val Lys His Phe Lys
        435                 440                 445

Ser Ser Thr Gly Asn Lys Leu Gly Val Glu Glu Lys Val Arg Arg Thr
    450                 455                 460

Leu Ala Ile His Gly Glu Ala Ser Lys Tyr Asp Asn Lys Tyr Val Ser
465                 470                 475                 480

Trp Lys Gly